# EUROPEAN PATENT SPECIFICATION

(11) **EP 1 485 060 B1**
(45) Date of publication and mention of the grant of the patent: **10.04.2013**
(21) Application number: 03745081.4
(22) Date of filing: 27.02.2003
(51) Int. Cl.: A61Q 1/00, A61Q 1/02

(54) **PERSONAL CARE COMPOSITIONS COMPRISING SOLID PARTICLES ENTRAPPED IN A GEL NETWORK**
HYGIENEZUSAMMENSETZUNGEN MIT FESTEN TEILCHEN IN EINEM GELNETZ
COMPOSITIONS DE SOINS PERSONNELS COMPRENANT DES PARTICULES SOLIDES PIEGEES DANS UN RESEAU DE TYPE GEL

(30) Priority: 18.03.2002 US 100637
(43) Date of publication of application: 15.12.2004
(73) Proprietor: THE PROCTER & GAMBLE COMPANY, Cincinnati, OH 45202 (US)
(72) Inventor: ADAMS, Christine, Helga, Egham, Surrey TW20 8JZ (GB); BROWNE, Yvonne, Bridget, Bagshot, Surrey GU19 5QY (GB); KALLA, Karen, Kay, Cincinnati, OH 45226 (US); MORRISSEY, Christopher, Todd, Mason, OH 45040 (US); MOTLEY, Curtis, Bobby, Hamilton, OH 45011 (US); STEPHENS, Alison, Fiona, Cookham, Berkshire SL6 9LA (GB); SUNKEL, Jorge, Max, Cincinnati, OH 45241 (US)
(74) Representative: Engisch, Gautier
(86) International application number: PCT/US2003/005975
(87) International publication number: WO 2003/080005

(56) References cited:
- EP-A- 0 848 029
- EP-A- 0 972 512
- EP-A- 1 020 494
- EP-A- 1 048 686
- EP-A- 1 057 872
- US-A- 5 066 485
- US-A- 5 234 711
- TAIZO MIYOSHI: 'Surface Treatment Technology for Cosmetics', [Online] April 2001, Retrieved from the Internet: <URL:http://www.miyoshikasei.com/eng/images /ohp.pdf.PDF> [retrieved on 2010-10-04]

## Description

### FIELD OF INVENTION

The present invention relates to personal care compositions suitable for use on mammalian skin. These compositions comprise a three-dimensional polymeric network that includes a polymer in which solid particles are entrapped and a solvent in which the polymer is dispersed. These compositions are intended to deliver even distribution of pigments and other solid particles to the skin or to like surfaces.

### BACKGROUND

A typical problem experienced in the application of color cosmetics to the skin is the difficulty associated with controlling the deposition of solid particles, particularly colorants, in the product due to the random dispersion of the particles in the composition. Oftentimes, these particles agglomerate within the product and cause clumping within the product as well as upon application to the skin. The ability to control the deposition is desirable as this would allow different finished looks to be created by the formulator.

One way of remedying this problem is to trap the colorants on or in a substrate, which is typically a rather hard material. Two examples of this solution include PMMA beads onto which iron oxides can be physically hammered into the surface and talc/mica platelets where a layer of titanium dioxide is coated on the surface. This solution, however, is somewhat limited to the use of relatively small substrate particles since larger particles tend to feel unpleasant upon application to the skin.

US Patent 5,066,485 to Brieva et al., issued November 19, 1991 and the internet article by Taizo Miyoshi with the title "Surface Treatment Technology for Cosmetics" dated April 2001 and retrievable on October 04, 2010 at http://www.miyoshikasei.com/eng/images/ohp.pdf.PDF disclose discrete solid colorant particles, whereby each particle is covalently coated with crosslinked organopolysiloxanes.

Furthermore, it is widely known that various cosmetic compositions are provided to simulate natural skin color, for example as foundations, or to provide accessory color, for example as blushes, eye shadows, lipsticks and the like. Additionally, in an attempt to revitalize and duplicate the natural and original properties of skin, various cosmetic compositions have also been developed, including, for example, foundations and concealers. Typically, the cosmetic compositions are applied to the skin to cover imperfections and/or simulate healthy or natural-looking skin.

Many current cosmetic compositions, however, typically fail to depict the color variation present in skin. As a result, the cosmetic compositions appear dull and unrealistic such that an unnatural skin look and/or color tone is achieved. Accordingly, there is also a need for providing cosmetic compositions that substantially represents a desired skin tone variation once applied to the skin. Additionally, it is desirable for these compositions to have enhanced color and to provide sufficient coverage once applied to skin.

Applicants, however, have overcome the limitations in solving the problem of solid particle e.g., pigment, agglomeration within a cosmetic composition. Applicants have found that the permanent entrapment of disperse colorants or solids within a polymeric network is key. In particular, this entrapment occurs during the polymerization process. This solution yields two major advantages. First, upon application to the skin of compositions containing this entrapment vehicle, the colorants and/or solids deposit while in the gel network, thereby allowing the consumer to determine the amount of pigment/solid, e.g., color, that is suitable and hence its control deposition upon the skin. Secondly, this solution allows the use of substantially larger substrate particles than typically seen since the three dimensional gel polymeric network in and of itself feels smooth and the inclusion of the entrapped colorants/solids seems to minimally affect the smooth feel such that a draggy feel is not experienced by the consumer. Thirdly, this solution permits for sufficient coverage of skin while also providing the desired look, whether natural or more dramatic.

These and other aspects of the present invention will become more readily apparent from consideration of the following summary and detailed description.

### SUMMARY OF THE INVENTION

In accordance with the present invention there is provided a personal care composition as defined in the appended claims.

The present invention relates to a personal care composition comprising a three dimensional gel polymeric network comprising:
a. a polymer;
b. one or more solid particles that are entrapped within said polymer during polymerization of said polymer; and
c. a solvent in which said polymer is dispersed.

An alternative embodiment of this invention relates to a personal care composition comprising:
a. a three dimensional gel polymeric network comprising:
   i. a polymer;
   ii. at least one first colorant that is entrapped within said polymer during polymerization of said polymer;
   iii. a solvent in which said polymer is dispersed; and
b. at least one second colorant that is substantially similar to said first colorant wherein said at least one second colorant is dispersed within said composition but is not entrapped in said polymer and is separate and distinct from said network.

Additionally, the present invention relates to a personal care composition comprising:
a. a three dimensional gel polymeric network comprising:
   i. a polymer;
   ii. at least one first colorant that is entrapped within said polymer during polymerization of said polymer;
   iii. a solvent in which said polymer is dispersed; and
b. at least one second colorant that is substantially different in color from said first colorant wherein said second colorant is dispersed within said composition but is [i] not entrapped in said polymer and is separate and distinct from said network.

In a preferred embodiment of the invention the personal care composition is provided as an emulsion selected from the group consisting of water-in-oil emulsions, oil-in-water emulsions, water in-silicone emulsions, and combinations thereof.

### DETAILED DESCRIPTION OF THE INVENTION

As used herein, "personal care products" means any color cosmetic, hair, skin care product and "personal care composition" refers to the formulation included in the personal care product that confers the desired benefit. The term "cosmetic" or "make-up" refers to products that leave color on the face, including foundation, blacks and browns, i.e., mascara, concealers, eye liners, brow colors, eye shadows, blushers, lip colors, powders, solid emulsion compact, and so forth. "Skin care products" are those used to treat or care for, or somehow moisturize, improve, or clean the skin. Products contemplated by the phrase "skin care products" include, but are not limited to, adhesives, bandages, toothpaste, anhydrous occlusive moisturizers, antiperspirants, deodorants, personal cleansing products, powder laundry detergent, fabric softener towels, occlusive drug delivery patches, nail polish, powders, tissues, wipes, hair conditioners-anhydrous, shaving creams and the like. The term "foundation" refers to liquid, creme, mousse, pancake, compact, concealer or like product created or reintroduced by cosmetic companies to even out the overall coloring of the skin. Foundation is manufactured to work better over moisturized and/or oiled skin. As used herein, "excess moisture" means an undesirable and/ or unhealthy level of bodily fluids deposited on human skin.

The tenn "ambient conditions" as used herein refers to surrounding conditions under about one atmosphere of pressure, at about 50% relative humidity, and at about 25°C, unless otherwise specified.

As used herein the term "comprising" means that the composition can contain other ingredients which are compatible with the composition and which preferably do not substantially disrupt the compositions of the present invention. The term encompasses the terms "consisting of" and "consisting essentially of".

Unless otherwise indicated, all percentages and ratios used herein are by weight of the total composition. All weight percentages, unless otherwise indicated, are on an actives weight basis. All measurements made are at 25°C, unless otherwise designated.

### Polymer

An essential component of the present invention is a polymer that is a polysiloxane that is a crosslinked organopolysiloxane polymer gel networks. For instance, particularly well-suited crosslinked organopolysiloxane polymer gel networks are formed from polymerization of an epoxy functional organosiloxane in the presence of an acid catalyst.

The crosslinked organopolysiloxane polymer gel networks employed as polymers in the present compositions may be derived in a number of ways, i.e., formed from various starting materials. Suitable polymer gel networks include addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane; condensation-curing organopolysiloxane compositions which cure in the presence of an organotin compound or a titanate ester, by a condensation reaction between an hydroxyl-terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation, or electron beams.

Addition reaction-curing organopolysiloxane compositions are preferred for their rapid curing rates and excellent uniformity of curing. A particularly preferred addition reaction-curing organopolysiloxane composition is prepared from:
(A) an organopolysiloxane having at least 2 lower alkenyl groups in each molecule;
(B) an organopolysiloxane having at least 2 silicon-bonded hydrogen atoms in each molecule; and
(C) a platinum-type catalyst.

With regard to the above, component (A) is the basic component of the silicone polymer gel network-generating organopolysiloxane, and curing proceeds by the addition reaction of this component with component (B) under catalysis by component (C). This component (A) must contain at least 2 silicon-bonded lower alkenyl groups in each molecule; an excellent cured product will not be obtained at fewer than two lower alkenyl groups because a network structure will not be formed. Said lower alkenyl groups are exemplified by vinyl, allyl, and propenyl. While the lower alkenyl groups can be present at any position in the molecule, their presence at the molecular terminals is preferred. The molecular structure of this component may be straight chain, branched straight chain, cyclic, or network, but a straight chain, possibly slightly branched, is preferred. The molecular weight of the component is not specifically restricted, and thus the viscosity may range from low viscosity liquids to very high viscosity gums. In order for the cured product to be obtained in the form of the rubbery polymer gel network, it is preferred that the viscosity at 25°C be at least 100 centistokes. These organopolysiloxanes are exemplified by methylvinylsiloxanes, methylvinylsiloxane-dimethylsiloxane copolymers, dimethylvinylsiloxy-terminated dimethylpolysiloxanes, dimethylvinylsiloxy-terminated dimethylsiloxane-methylphenylsiloxane copolymers, dimethylvinylsiloxy-terminated dimethylsiloxane-diphenylsiloxane-methylvinylsiloxane copolymers, trimethylsiloxy-terminated dimethylsiloxane-methylvinylsiloxane copolymers, trimethylsiloxy-terminated dimethylsiloxane-methylphenylsiloxane-methylvinylsiloxane copolymers, dimethylvinylsiloxy-terminated methyl(3,3,3-trifluoropropyl) polysiloxanes, and dimethylvinylsiloxy-terminated dimethylsiloxane-methyl(3,3,-trifluoropropyl)siloxane copolymers.

Component (B) is an organopolysiloxane having at least 2 silicon-bonded hydrogen atoms in each molecule and is a crosslinker for component (A). Curing proceeds by the addition reaction of the silicon-bonded hydrogen atoms in this component with the lower alkenyl groups in component (A) under catalysis by component (C). This component (B) must contain at least 2 silicon-bonded hydrogen atoms in each molecule in order to function as a crosslinker. Furthermore, the sum of the number of alkenyl groups in each molecule of component (A) and the number of silicon-bonded hydrogen atoms in each molecule of component (B) is to be at least 5. Values below 5 should be avoided because a network structure is then essentially not formed.

No specific restriction exists on the molecular structure of this organopolysiloxane starting material component, and it may be any of straight chain, branch-containing straight chain, cyclic, etc. The molecular weight of this component is not specifically restricted, but it is preferred that the viscosity at 25°C be 1 to 50,000 centistokes in order to obtain good miscibility with component (A). It is preferred that this component be added in a quantity such that the molar ratio between the total quantity of silicon-bonded hydrogen atoms in the instant component and the total quantity of all lower alkenyl groups in component (A) falls within the range of 1.5:1 to 20:1. It is difficult to obtain good curing properties when this molar ratio falls below 0.5:1. When 20:1 is exceeded, there is a tendency for the hardness to increase to high levels when the cured product is heated. Furthermore, when an organosiloxane containing substantial alkenyl is supplementarily added for the purpose of; for example, reinforcement, it is preferred that a supplemental addition of the instant SiH-containing component be made in a quantity offsetting these alkenyl groups. This component is concretely exemplified by trimethylsiloxy-terminated methylhydrogenpolysiloxanes, trimethylsiloxy-terminated dimethylsiloxane-methylhydrogensiloxane copolymers, and dimethylsiloxane-methylhydrogen-siloxane cyclic copolymers.

Component (C) is a catalyst of the addition reaction of silicon-bonded hydrogen atoms and alkenyl groups, and is concretely exemplified by chloroplatinic acid, possibly dissolved in an alcohol or ketone and this solution optionally aged, chloroplatinic acid-olefin complexes, chloroplatinic acid-alkenylsiloxane complexes, chloroplatinic acid-diketone complexes, platinum black, and carrier-supported platinum.

This component is added preferably at 0.1 to 1,000 weight parts, and more preferably at 1 to 100 weight parts, as platinum-type metal proper per 1,000,000 weight parts of the total quantity of components (A) plus (B). Other organic groups which may be bonded to silicon in the organopolysiloxane forming the basis for the above-described curable organopolysiloxane compositions are, for example, alkyl groups such as methyl, ethyl, propyl, butyl, and octyl; substituted alkyl groups such as 2-phenylethyl, 2-phenylpropyl, and 3,3,3-trifluoropropyl; aryl groups such as phenyl, tolyl, and xylyl; substituted aryl groups such as phenylethyl; and monovalent hydrocarbon groups substituted by, for example, the epoxy group, the carboxylate ester group, the mercapto group, etc.

An example of the production of the organopolysiloxane polymer gel network powder includes the process in which an organopolysiloxane composition as described above (additional-curable, condensation-curable, or peroxide-curable) is mixed with water in the presence of a surfactant (nonionic, anionic, cationic, or amphoteric), and, after mixing to homogeneity in a homomixer, colloid mill, homogenizer, propeller mixer, etc., this is cured by discharge into hot water (temperature at least 50°C) and is then dried; the organopolysiloxane composition (addition-curable, condensation-curable, or peroxide-curable) is cured by spraying it directly into a heated current; the powder is obtained by curing a radiation-curable organopolysiloxane composition by spraying it under high energy radiation; the organopolysiloxane composition (addition-curable, condensation-curable, peroxide-curable) or high energy-curable organopolysiloxane composition is cured, the latter by high energy radiation, and the product is then pulverized using a known pulverizer such as, for example, a ball mill, atomizer, kneader, roll mill, etc., to thereby form the powder. Suitable organopolysiloxane polymer network powders include vinyl dimethicone/methicone silesquioxane crosspolymers like Shin-Etsu's KSP-100, KSP-101, KSP-102, KSP-103, KSP-104, KSP-105, hybrid silicone powders that contain a fluoroalkyl group like Shin-Etsu's KSP-200, and hybrid silicone powders that contain a phenyl group such as Shin-Etsu's KSP-300; and Dow Coming's DC 9506.

Preferred organopolysiloxane compositions are dimethicone/vinyl dimethicone crosspolymers. Such dimethicone/vinyl dimethicone crosspolymers are supplied by a variety of suppliers including Dow Coming (DC 9040 and DC 9041), General Electric (SFE 839), Shin Etsu (KSG-15, 16, 18 [dimethicone /phenyl vinyl dimethicone crosspolymer] and KSG-21 [dimethicone copolyol crosspolymer]), Grant Industries (Gransil™ line of materials), lauryl dimethicone/vinyl dimethicone crosspolymers supplied by Shin Etsu (e.g., KSG-41, KSG-42, KSG-43, and KSG-44), lauryl dimethicone/ dimethicone copolyol crosspolymers also supplied by Shin-Etsu (e.g., KSG-31, KSG-32, KSG-33, and KSG-34). Additional polymers from Shin-Etsu which are suitable fro use in the present invention include KSG-210, -310, 320, 330, and 340. Crosslinked organopolysiloxane polymer gel networks useful in the present invention and processes for making them are further described in US Patent 4,970,252 to Sakuta et al., issued November 13, 1990; US Patent 5,760,116 to Kilgour et al., issued June 2, 1998; US Patent 5,654,362 to Schulz, Jr. et al. issued August 5, 1997; and Japanese Patent Application JP 61-18708, assigned to Pola Kasei Kogyo KK.

Another silicone-based polymer that is suitable for inclusion into the presently claimed compositions is a polyethersiloxane block copolymer network comprising one or more polyether blocks, each comprising i) two or more structural units of the formula -R¹O- wherein each R¹ is independently a divalent hydrocarbon radical or R², wherein R² is a trivalent hydrocarbon radical, and ii) one or more polysiloxane blocks, each comprising two or more structural units of the formula -R³₂ SiO_{2/2}- wherein each R³ is independently a monovalent hydrocarbon radical or R², and wherein at least one polyether block of the copolymer network is bonded to at least one polysiloxane block of the copolymer network by a link according to formula 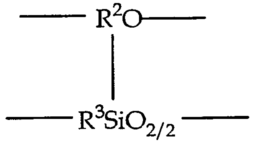 wherein the R2O unit of this formula is a unit of the at least one polyether block and the R²R³SiO_{2/2} unit of the structure of this formula is a unit of the at least one polysiloxane unit. This copolymer network is described in further detail in copending U.S. application Serial No. 09/592,193, filed on June 12, 2000 in the name of Kilgour et al.

The compositions of the present invention comprise a combination of emulsifying and non-emulsifying crosslinked organopolysiloxane polymer gel networks as the polymer. The term "non-emulsifying," as used herein, defines a crosslinked organopolysiloxane polymer gel network from which polyoxyalkylene units are absent unless formed as a result of a reaction between reactive sites such as epoxy groups that are substituted in polymer chains to act as crosslinkers. The term "emulsifying," as used herein, means a crosslinked organopolysiloxane polymer gel network having at least one polyoxyalkylene unit that is not formed as a result of a reaction between reactive sites such as epoxy groups that are substituted in polymer chains to act as crosslinkers. Emulsifying crosslinked organopolysiloxane polymer gel network can notably be chosen from the crosslinked polymers described in US Patents 5,412,004 (issued 5/2/95); 5,837,793 (issued 11/17/98); and 5,811,487 (issued 9/22/98).

Particularly useful emulsifying polymer gel networks are polyoxyalkylene modified polymer gel networks formed from divinyl compounds, particularly siloxane polymers with at least two free vinyl groups, reacting with Si-H linkages on a polysiloxane backbone. Preferably, the polymer gel networks are dimethyl polysiloxanes crosslinked by Si-H sites on a molecularly spherical MQ resin.

The non-emulsifying crosslinked organopolysiloxane polymer gel networks of the present invention are preferably further processed by subjecting them to a high shear (approximately 5,000 psi) treatment in the presence of a solvent for the siloxane polymer gel network via a Sonolator at less than 10 passes. Sonolation achieves a resultant composition with polymer gel network average particle size ranging from at least about microns to about 200 microns, preferably from about 20 to about 100 microns, more preferably from about 25 to about 80 microns and most preferably from about 30 microns to about 65 microns as measured by the Horiba LA-910 (described below). When the compositions of the present invention are utilized for atypical cosmetic purposes, e.g., costume makeup or fashion-type makeup products, a wider particle size range would most likely be suitable. In this case, the particle of the polymers may range from about 20 microns to about 200 microns, preferably from about 30 to about 150 microns, more preferably from about 40 to about 95 microns and most preferably from about 50 microns to about 90 microns as measured by the Horiba LA-910 (described below). As used herein, the term "particle size" of the polymer gel network represents the polymer gel network particle size in its swelled state. By "swelled," as used herein, means that the polymer gel network particles have extended beyond their normal size and shape by virtue of their absorption of the solvent compound. Viscosity is best when ranging between above 20,000 (or above about 20,000) and about 6,000,000, preferably from about 25,000 to about 4,000,000, more preferably from about 30,000 to about 3,000,000, most preferably from about 40,000 to about 2,000,000, optimally about 60,000 to about 1,500,000 cps at 25°C as measured by a Brookfield LV Viscometer (size 4 bar, 60 rpm, 0.3 sec).

Preferably, the non-emulsifying crosslinked organopolysiloxane polymer gel networks do not undergo recycled processing. Without being limited by theory, recycled processing produces broad particle size distributions comprising particles larger or smaller than that necessary to achieve the skin feel benefits of the present invention. Specifically, gel balls often result from silicone polymer gel network particles larger than 200 microns while polymer gel network particles smaller than 10 microns reduce skin feel and viscosity benefits. Such particle size distributions result from a failure to ensure that all of the polymer gel network particle materials experience the same shear throughout the process. Typically, with recycling, only a portion of the particles experience shear before these sheared particles are returned to the process starting point and combined with the remaining un-sheared particles. Similarly, the next cycle begins with only a portion of this particle mixture experiencing before the newly sheared mixture particles are returned to the process starting point and combined with the remaining un-sheared particle mixture. Importantly, even after considerable recycling, some of the particles never actually experience shear while others experience a high degree of shear. The result is a particle size range, which encompasses particles both larger and smaller than those necessary to achieve the present invention.

In contrast, discrete pass processing, as alluded to above, ensures that all the particles experience shear as well as the same amount of shear with each run or pass. More specifically, no run or pass is completed until all the particles have experienced the same shear force. Consequently, the particle size distribution is narrower than that produced by "recycling" with respect to specific particle sizes. This results in a better balance between gel ball formation and viscosity as well as skin feel and viscosity.

The present compositions comprise from about 0.1% to about 15%, by weight of the composition, of the polymer. In preferred embodiments, the polymer is present in the composition in an amount of from about 2% to about 10%, by weight of the composition.

### Solid Particles

The compositions of the present invention further essentially comprise one or more solid particles that are entrapped within the polymer during polymerization of the polymer. As used herein "entrapped" means permanent inclusion of the solid particle within the interstices (or spaces) of the carrier matrix which is the polymer. The compositions of the present invention include solid particles that are selected from the group consisting of shine control agents, soft focus powders, sunscreen powders, colorants, filler powders, and combinations thereof. As used herein, "colorants" generally refer to a pigment, lake, toner, dye or other agent used to impart a color expression to a material. Furthermore, the compositions comprise from about 0.1% to about 15%, by weight of the polymer, of the solid particles, more preferably from about 5% to about 12%, and most preferably, from about 7% to about 10%.

There are no specific limitations as to the pigment, colorant or filler powders that can be used as the "solid particle" of the composition. Each may be a body pigment, inorganic white pigment, inorganic colored pigment, pearling agent, and the like. Specific examples are talc, mica, magnesium carbonate, calcium carbonate, magnesium silicate, aluminum magnesium silicate, silica, titanium dioxide, zinc oxide, red iron oxide, yellow iron oxide, black iron oxide, ultramarine, polyethylene powder, methacrylate powder, polystyrene powder, silk powder, crystalline cellulose, starch, titanated mica, iron oxide titanated mica, bismuth oxychloride, and the like.

Some of the colorants which can be used herein include, but are not limited to, D&C Yellow No. 7, D&C Red No. 36, FD&C Red No. 3, FD&C Red No. 4, D&C Orange No. 4, D&C Red No. 6, D&C Red No. 34, FD&C Yellow No. 6, D&C Red No. 33, FD&C Yellow No. 5, D&C Brown No. 1, D&C Red No. 17, FD&C Green No. 3, D&C Blue No. 4, D&C Yellow No. 8, D&C Orange No. 5, D&C Red No. 22, D&C Red No. 21, D&C Red No. 28, D&C Orange No. 11, D&C Yellow No. 10, D&C Violet No. 2, Ext. D&C Violet No. 2, D&C Green No. 6, D&C Green No. 5, D&C Red No. 30, D&C Green No. 8, D&C Red No. 7, FD&C Blue No. 1, D&C Yellow No. 7, D&C Red No. 27, D&C Orange No. 10, D&C Red No. 31, FD&C Red No. 40, D&C Yellow No. 11, Annatto extract, β carotene, guanine, carmine, aluminum powder, ultramarines, bismuth oxychloride, chromium oxide green, chromium hydroxide green, iron oxides, ferric ferrocyanide, manganese violet, titanium dioxide, zinc oxide, caramel coloring, mica, ferric ammonium ferrocyanide, dihydroxyacetone, guaiazulene, pyrophyllite, bronze powder, copper powder, aluminum stearate, calcium stearate, lactofavin, magnesium stearate, zinc stearate, capsanthin/capsorubin, bentonite, barium sulfate, calcium carbonate, calcium sulfate, carbon black, magnesium carbonate, colored silica, CI 10020, CI 11680, CI 15630, CI 15865, CI 16185, CI 16255, CI 16255, CI 45430, CI 69825, CI 73000, CI 73015, CI 74160, CI 75100, CI 77002, CI 77346, CI 77480, Brown, Russet and Sienna dyes. Additionally, lakes, composites or encapsulates of these colorants may also be used.

Lakes are either a pigment that is extended or reduced with a solid diluent or an organic pigment that is prepared by the precipitation of a water-soluble dye on an adsorptive surface, which usually is aluminum hydrate. There is uncertainty in some instances as to whether the soluble dye precipitates on the surface of the aluminum hydrate to yield a dyed inorganic pigment or whether it merely precipitates in the presence of the substrate. A lake also forms from precipitation of an insoluble salt from an acid or basic dye. Calcium and barium lakes are also used herein.

Additional pigments and filler powders include, but are not limited to, inorganic powders such as gums, chalk, Fuller's earth, kaolin, sericite, muscovite, phlogopite, synthetic mica, lepidolite, biotite, lithia mica, vermiculite, aluminum silicate, starch, smectite clays, alkyl and/or trialkyl aryl ammonium smectites, chemically modified magnesium aluminum silicate, organically modified montmorillonite clay, hydrated aluminum silicate, fumed aluminum starch octenyl succinate barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, metallic soap (zinc stearate, magnesium stearate, zinc myristate, calcium palmitate, and aluminum stearate), colloidal silicone dioxide, and boron nitride; organic powder such as polyamide resin powder (nylon powder), cyclodextrin, methyl polymethacrylate powder, copolymer powder of styrene and acrylic acid, benzoguanamine resin powder, poly(ethylene tetrafluoride) powder, and carboxyvinyl polymer, cellulose powder such as hydroxyethyl cellulose and sodium carboxymethyl cellulose, ethylene glycol monostearate; inorganic white pigments such as magnesium oxide. Other useful powders are disclosed in U.S. Patent 5, 688,831. These pigments and powders can be used independently or in combination.

Also useful herein as solid particles are pigment and/or dye encapsulates such nanocolorants from BASF and multi-layer interference pigments such as Sicopearls from BASF.

In preferred embodiments of the present invention at least one first colorant is entrapped within a polymer that is swollen in a fluid to form a gel network and at least one second colorant is contained within the same composition but outside of the gel network. In certain embodiments, the at least one second colorant is substantially similar to the at least one first colorant while in other embodiments the at least one second colorant is substantially different from the at least one first colorant. It is important to note that as described the present invention is intended to encompass mixtures of one or more colorants within the three dimensional gel network and a similar mixture of one or more colorants outside of the gel network but within the composition. In these embodiments, it is preferred that the at least one second colorant is a hydrophilic pigment that is included in the aqueous phase of the emulsion of the composition. As used herein, "substantially similar" means that the colors exhibited by the first and second colorants are almost the same color or shade as one another. On the other hand, "substantially different" refers to the first and second colorants being quite distinct from each other relative to their colors or shades. In certain embodiments there may be more than one three dimensional gel network in a composition wherein each gel network contains one or more colorants that are contained in the respective gel networks.

Dispersants may also be used in conjunction with the colors and pigments of the present invention. Examples of suitable dispersants include, but are not limited to, those described in U.S. Patent 5,688,493.

It is preferred that the pigments/powders are surface treated to provide added stability of color and ease of formulation. Hydrophobically treated pigments are more preferred, because they may be more easily dispersed in the solvent/oil phase. In addition, it may be useful to treat the pigments with a material that is compatible with a silicone phase. Particularly useful hydrophobic pigment treatments for use in water-in-silicone emulsions include polysiloxane treatments such as those disclosed in U.S. Patent 5,143,722. Also preferred are pigment/powders having a primary average particle size of from about 5 nm to about 100,000 nm, more preferably from about 50nm to about 5,000 nm, most preferably from about 100nm to about 1000 nm. Mixtures of the same or different pigment/powder having different particle sizes are also useful herein (e.g., incorporating a TiO2 having a primary particle size of from about 100 nm to about 400 nm with a TiO2 having a primary particle size of from about 10 nm to about 50 nm).

Certain shine control agents are suitable for use as solid particles in the present compositions. Those agents or particles include, but are not limited to, silicas, magnesium aluminum silicates, talc and sericite. Shine control agents to be used as solid particles in the present compositions are selected from silicates or carbonates that are formed by reaction of a carbonate or silicate with the alkali (IA) metals, alkaline earth (IIA) metals, or transition metals, and silicas (silicon dioxide). Preferred shine control agents are selected from the group consisting of calcium silicates, amorphous silicas, calcium carbonates, magnesium carbonates, zinc carbonates, and combinations thereof. Some specific examples of the silicates and carbonates useful in this present invention are more fully explained in Van Nostrand Reinhold's Encyclopedia of Chemistry, 4th Ed. Pp. 155, 169, 556, and 849 (1984).

Synthetic versions of the shine control agents, particularly silicates, are preferred. Examples of synthetic silicates useful in the present invention are Hubersorb 250® or Hubersorb 600®, available from J. M. Huber.

Shine control agents that primarily comprise silicas are suitable solid particles as well. Silicas that are useful herein may be in the form of microspheres and/ or ellipsoids, as they have been found to contribute good skin feel characteristics in addition to efficient moisture absorption. Silica ellipsoids useful in the present invention are available from DuPont as ZELEC Sil and Kobo as Silica Shells. Silica microspheres are available from Kobo as MSS-500, MSS500/3, MSS-500H, MSS500/3N, MSS-500N and MSS 500/ 3N; Presperse as Spheron L1500, Spheron P1500. Fumed versions of silica can also be used with Aerosil from Degussa and Cab-O-Sil from Cabot both being particularly useful.

Amongst the silicate series, magnesium aluminum silicates are useful, in particular Sebumase, available from Miyoshi Kasei.

Starch-based materials may also be used as shine control agents but do not form part of the present invention. Useful examples are Natrosorb W and Natrosorb HFW, DryFlo plus and DryFlo AF pure from National Starch and Chemical Company.

Also found to be useful are methacrylate-based polymeric materials. However, they also do not form part of the present invention. They can be used either in conjunction with a dimethicone copolymer or as methacrylate-based copolymers. Specifically, useful examples are: Microsponge 5640 w. Glycerin, Polytrap 6603 available from Enhanced Derm technologies; DSPCS-I2 series and SPCAT-I2 from Kobo; Poly-Pore 200 series from Amcol.

The compositions of the present invention may contain solid particles that are optionally spherical. When the solid particles are spherical, it is preferred that they have an average particle size diameter of 10 or greater, preferably greater than 15, more preferably greater than 20 microns. The particle diameter is understood to be that of elementary or primary particles.

Preferred spherical solid particles include, but are not limited, to polymeric particles chosen from the methylsilsesquioxane resin microspheres such as for example those sold by Toshiba silicone under the name Tospearl 145A; KSP powders, X-100, X-200, and X-300 powders from Shin-Etsu; microspheres of polymethylmethacrylates such as those sold by Seppic under the name Micropearl M 100; the spherical solid particles of crosslinked polydimethylsiloxanes, especially such as those sold by Dow Coming Toray Silicone under the name Trefil E 506C or Trefil E 505C, spherical solid particles of polyamide and more specifically Nylon 12, especially such as those sold by Atochem under the name Orgasol 2002D Nat C05, polystyerene microspheres such as for example those sold by Dyno Particles under the name Dynospheres, ethylene acrylate copolymer sold by Kobo under the name FloBead EA209 and mixtures thereof. Also found to be useful is Ronasphere LDP from Kobo, Inc.

Without being limited by theory it is believed that entrapping the solid particles within the polymer during the polymerization process allows for the unagglomerated incorporation of the particles into the resultant gel network and finally into the composition. This ultimately facilitates the even spreading of the compositions of the present invention onto a surface with little to no agglomeration of the solid particles on the surface or even in the packaging.

### Solvent for the Polymer

The compositions of the present invention comprise a solvent for the polymer described above. The solvent, when combined with the polymer, serves to suspend and swell the polymer to provide an elastic, three-dimensional gel network or matrix. The solvent for the polymer is liquid under ambient conditions, and preferably has a low viscosity to provide for improved spreading on the skin.

Concentrations of the solvent in the cosmetic compositions of the present invention will vary primarily with the type and amount of polymer employed. Preferred concentrations of the solvent are from about 1% to about 90%, preferably from about 10% to about 60%, more preferably from about 15% to about 40%, by weight of the composition.

The solvent for the polymer comprises one or more liquid carriers suitable for topical application to human skin. These liquid carriers may be organic, silicone-containing or fluorine-containing, volatile or non-volatile, polar or non-polar, provided that the liquid carrier forms a three-dimensional gel network when combined with the polymer at a temperature of from about 28°C to about 250°C, preferably from about 28°C to about 100°C, preferably from about 28°C to about 78°C. The solvent for the crosslinked siloxane polymer gel network preferably has a solubility parameter of from about 3 to about 13 (cal/cm³)^{0.5}, more preferably from about 5 to about 11 (cal/cm³)^{0.5}, most preferably from about 5 to about 9 (cal/cm³)^{0.5}. Solubility parameters for the liquid carriers or other materials, and means for determining such parameters, are well known in the chemical arts. A description of solubility parameters and means for determining them are described by C. D. Vaughan, "Solubility Effects in Product, Package, Penetration and Preservation" 103 Cosmetics and Toiletries 47-69, October 1988; and C. D. Vaughan, "Using Solubility Parameters in Cosmetics Formulation", 36 J. Soc. Cosmetic Chemists 319-333, September/October, 1988.

The solvent preferably includes volatile, non-polar oils; non-volatile, relatively polar oils; non-volatile, non-polar oils; and non-volatile paraffinic hydrocarbon oils; each discussed more fully hereinafter. The term "non-volatile" as used herein refers to materials which exhibit a vapor pressure of no more than about 0.2 mm Hg at 25°C at one atmosphere and/or to materials that have a boiling point at one atmosphere of at least about 300°C. The term "volatile" as used herein refers to all materials that are not "non-volatile" as previously defined herein. The phrase "relatively polar" as used herein means more polar than another material in terms of solubility parameter; i.e., the higher the solubility parameter the more polar the liquid. The term "non-polar" typically means that the material has a solubility parameter below about 6.5 (cal/cm³)^{0.5}.

### 1. Non-polar, Volatile Oils

The non-polar, volatile oil tends to impart highly desirable aesthetic properties to the compositions of the present invention. Consequently, the non-polar, volatile oils are preferably utilized at a fairly high level. Non-polar, volatile oils particularly useful in the present invention are selected from the group consisting of silicone oils; hydrocarbons; and mixtures thereof. Such non-polar, volatile oils are disclosed, for example, in Cosmetics, Science, and Technology, Vol. 1, 27-104 edited by Balsam and Sagarin, 1972. The non-polar, volatile oils useful in the present invention may be either saturated or unsaturated, have an aliphatic character and be straight or branched chained or contain alicyclic or aromatic rings. Examples of preferred non-polar, volatile hydrocarbons include polydecanes such as isododecane and isodecane (e.g., Permethyl-99A which is available from Presperse Inc.) and the C7 - C8 through C12 - C15 isoparaffins (such as the Isopar Series available from Exxon Chemicals). Non-polar, volatile liquid silicone oils are disclosed in U.S. Patent 4,781,917. Additionally, a description of various volatile silicones materials is found in Todd et al., "Volatile Silicone Fluids for Cosmetics", Cosmetics and Toiletries, 91:27-32 (1976). Particularly preferred volatile silicone oils are selected from the group consisting of cyclic volatile silicones corresponding to the formula: 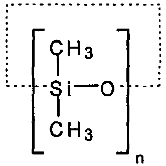 wherein n is from about 3 to about 7; and linear volatile silicones corresponding to the formula:

(CH₃)₃ Si-O-[Si(CH₃)₂-O]ₘ --Si(CH₃)₃

wherein m is from about 1 to about 7. Linear volatile silicones generally have a viscosity of less than about 5 centistokes at 25°C., whereas the cyclic silicones have viscosities of less than about 10 centistokes at 25°C. Highly preferred examples of volatile silicone oils include cyclomethicones of varying viscosities, e.g., Dow Coming 200, Dow Coming 244, Dow Coming 245, Dow Corning 344, and Dow Coming 345, (commercially available from Dow Coming Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.).

### 2. Relatively Polar, Non-volatile oils

The non-volatile oil is "relatively polar" as compared to the non-polar, volatile oil discussed above. Therefore, the non-volatile co-solvent is more polar (i.e., has a higher solubility parameter) than at least one of the non-polar, volatile oils. Relatively polar, non-volatile oils potentially useful in the present invention are disclosed, for example, in Cosmetics, Science, and Technology, Vol. 1, 27-104 edited by Balsam and Sagarin, 1972; U.S. Patents 4,202,879 issued to Shelton on May 13, 1980; and 4,816,261 issued to Luebbe et al. on Mar. 28, 1989. Relatively polar, non-volatile oils useful in the present invention are preferably selected from the group consisting of silicone oils; hydrocarbon oils; fatty alcohols; fatty acids; esters of mono and dibasic carboxylic acids with mono and polyhydric alcohols; polyoxyethylenes; polyoxypropylenes; mixtures of polyoxyethylene and polyoxypropylene ethers of fatty alcohols; and mixtures thereof. The relatively polar, non-volatile co-solvents useful in the present invention may be either saturated or unsaturated, have an aliphatic character and be straight or branched chained or contain alicyclic or aromatic rings. More preferably, the relatively polar, non-volatile liquid co-solvent are selected from the group consisting of fatty alcohols having from about 12-26 carbon atoms; fatty acids having from about 12-26 carbon atoms; esters of monobasic carboxylic acids and alcohols having from about 14-30 carbon atoms; esters of dibasic carboxylic acids and alcohols having from about 10-30 carbon atoms; esters of polyhydric alcohols and carboxylic acids having from about 5-26 carbon atoms; ethoxylated, propoxylated, and mixtures of ethoxylated and propoxylated ethers of fatty alcohols with from about 12-26 carbon atoms and a degree of ethoxylation and propoxylation of below about 50; and mixtures thereof. More preferred are propoxylated ethers of C14 - C18 fatty alcohols having a degree of propoxylation below about 50, esters of C2 -C8 alcohols and C12-C26 carboxylic acids (e.g. ethyl myristate, isopropyl palmitate), esters of C12-C26 alcohols and benzoic acid (e.g. Finsolv TN supplied by Finetex), diesters of C2-C8 alcohols and adipic, sebacic, and phthalic acids (e.g., diisopropyl sebacate, diisopropyl adipate, di-n-butyl phthalate), polyhydric alcohol esters of C6 -C26 carboxylic acids (e.g., propylene glycol dicaprate/dicaprylate, propylene glycol isostearate); and mixtures thereof. Even more preferred are branched-chain aliphatic fatty alcohols having from about 12-26 carbon atoms. Even more preferred are isocetyl alcohol, octyldecanol, octyldodecanol and undecylpentadecanol; and most preferred is octyldodecanol. Such preferred aliphatic fatty alcohols are particularly useful in combination with the volatile liquid silicone oils discussed herein to adjust the average solubility of the solvent.

### 3. Non-polar, Non-volatile oils

In addition to the liquids discussed above, the solvent for the crosslinked siloxane polymer gel network may optionally include non-volatile, non-polar oils. Typical non-volatile, non-polar emollients are disclosed, for example, in Cosmetics, Science, and Technology, Vol. 1, 27-104 edited by Balsam and Sagarin, 1972; U.S. Patents 4,202,879 and 4,816,261. The non-volatile oils useful in the present invention are essentially non-volatile polysiloxanes, paraffinic hydrocarbon oils, and mixtures thereof. The polysiloxanes useful in the present invention selected from the group consisting of polyalkylsiloxanes, polyarylsiloxanes, polyalkylarylsiloxanes, poly-ethersiloxane copolymers, and mixtures thereof. Examples of these include polydimethyl siloxanes having viscosities of from about 1 to about 100,000 centistokes at 25° C. Among the preferred non-volatile silicone emollients useful in the present compositions are the polydimethyl siloxanes having viscosities from about 2 to about 400 centistokes at 25°C. Such polyalkylsiloxanes include the Viscasil series (sold by General Electric Company) and the Dow Coming 200 series (sold by Dow Coming Corp.). Polyalkylarylsiloxanes include polymethylphenyl siloxanes having viscosities of from about 15 to about 65 centistokes at 25° C. These are available, for example, as SF 1075 methyl-phenyl fluid (sold by General Electric Company) and 556 Cosmetic Grade Fluid (sold by Dow Coming Corp.). Useful polyethersiloxane copolymers include, for example, a polyoxyalkylene ether copolymer having a viscosity of about 1200 to 1500 centistokes at 25°C. Such a fluid is available as SF1066 organosilicone surfactant (sold by General Electric Company). Polysiloxane ethylene glycol ether copolymers are preferred copolymers for use in the present compositions.

Non-volatile paraffmic hydrocarbon oils useful in the present invention include mineral oils and certain branched-chain hydrocarbons. Examples of these fluids are disclosed in U.S. Patent 5,019,375. Preferred mineral oils have the following properties:
(1) viscosity from about 5 centistokes to about 70 centistokes at 40°C;
(2) density between about 0.82 and 0.89 g/cm³ at 25°C;
(3) flash point between about 138°C and about 216° C; and
(4) carbon chain length between about 14 and about 40 carbon atoms.
Preferred branched chain hydrocarbon oils have the following properties:
(1) density between about 0.79 and about 0.89 g/cm3 at 20°C.
(2) boiling point greater than about 250° C; and
(3) flash point between about 110°C and about 200°C.
Particularly preferred branched-chain hydrocarbons include Permethyl 103 A, which contains an average of about 24 carbon atoms; Permethyl 104A, which contains an average of about 68 carbon atoms; Permethyl 102A, which contains an average of about 20 carbon atoms; all of which may be purchased from Permethyl Corporation; and Ethylflo 364 which contains a mixture of 30 carbon atoms and 40 carbon atoms and may be purchased from Ethyl Corp.

Additional solvents useful herein are described in US Patent 5,750,096.

### OPTIONAL INGREDIENTS

### Additional Polymers

The compositions of the present invention may optionally include polymers outside of those that are entrapped or contained in the three-dimensional gel network. For instance, they may be included to impart an improvement in feel to the compositions or simply to thicken the compositions. Suitable polymers include crosslinked polymers like phenol-formaldehyde resins; polyesters (i.e., saturated, unsaturated, and alkyds); epoxy resins that are cured with amines, amine-terminated polyamides, amidoamines, acid catalysts, tertiary amine catalysts, carboxylic acids, anhydrides, and phenols; isocyanates; vinyl esters; polypeptides; monovinylmultivinyl crosslinked polymers; urethanes; polyurethanes; amino resins; acrylics with functional groups like hydroxyls, glycidyls, carboxylics, isocyanates, oxazolidines, aziridines, activated esters, and vinyl acrylics; acetylene derivatives like acetylene-terminated polyimides, polyphenylene quinoxolines, diaryl acetylenes; polyimides, cyanates; nitriles; polysiloxanes, natural polymers (e.g., starches), cellulosics, and unsaturated hydrocarbons. Additionally, other linear, random branched, star, or unpigmented polymers and gels may also be included in the compositions of the present invention.

### Film Forming Agents

Film forming agents may be optionally included in the compositions of the present invention to aid film substantivity and adhesion to the skin. Improving the long wear and non-transfer performance of the present compositions is quite desirable. Water-soluble, water insoluble, and water dispersible film forming agents can be used in the internal and external phases of the present compositions to give the desired end benefit.

Preferably, the compositions comprise from about 0% to about 20%, more preferably, from about 0.1% to about 10%, and most preferably, from about 0.1% to about 5%, by weight of the composition, of the film-forming agent.

Suitable film forming agents include:
1) organic silicone resins, fluorinated silicone resins, copolymers of organic silicone resins, e.g., trimethylsiloxysilicate from GE (SR1000), GE's copolymers of silicone resins, e.g., SF1318 (silicone resin and an organic ester of isostearic acid copolymer) and CF1301 (silicone resin and alpha methyl styrene copolymer), Dow Coming's pressure sensitive adhesives - copolymers of silicone resins and various PDMS's (BIO-PSA series); and
2) acrylic and methacrylic polymers and resins, silicone-acrylate type copolymers and fluorinated versions of, including - silicones plus polymer SA70 from 3M, KP545 from Shin-Etsu, alkyl-acrylate copolymers, e.g., KP 561 and 562 from Shin-Etsu;
3) decene/butene copolymer from Collaborative Labs;
4) polyvinyl based materials, e.g., PVP, PVP/VA, including Antaron/Ganex from ISP (PVP/Triacontene copolymer), Luviskol materials from BASF;
5) polyurethanes, e.g., the Polyderm series from Alzo including but not limited to Polyderm PE/PA, Polyderm PPI-SI-WS, Polyderm PPI-GH, Luviset P.U.R. from BASF;
6) polyquaternium materials, e.g., Luviquat series from BASF
7) acrylates copolymers and acrylates/acrylamide copolymers, e.g., Luvimer and Ultrahold series, both available from BASF;
8) styrene based materials; and
9) chitosan and chitosan based materials including cellulose and cellulose-based materials.

### Skin Conditioning Agent

Optionally, the compositions of the present invention can further comprise a skin-conditioning agent. These agents may be selected from humectants, exfoliants or emollients.

Humectants are polyhydric alcohols intended for moisturizing, reducing scaling and stimulating removal of built-up scale from the skin. Typical polyhydric alcohols include polyalkylene glycols and more preferably alkylene polyols and their derivatives. Illustrative are propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin and mixtures thereof. Most preferably the humectant is glycerin. When present, amounts of humectant may range anywhere from 1 to 50%, preferably from 10 to 40%, optimally from 25 to 35% by weight of the composition.

Exfoliants according to the present invention may be selected from C2-C30 alpha-hydroxycarboxylic acids, beta-hydroxycarboxylic acids and salts of these acids. Most preferred are glycolic, lactic and salicylic acids and their ammonium salts. Amounts of the exfoliants may range from 1 to 15%, preferably from 2 to 10% by weight.

A wide variety of C2-C30 alpha-hydroxycarboxylic acids may be employed. Suitable examples of which include:
alpha -hydroxyethanoic acid
alpha -hydroxypropanoic acid
alpha -hydroxyhexanoic acid
alpha -hydroxyoctanoic acid
alpha -hydroxydecanoic acid
alpha -hydroxydodecanoic acid
alpha -hydroxytetradecanoic acid
alpha -hydroxyhexadecanoic acid
alpha -hydroxyoctadecanoic acid
alpha -hydroxyeicosanoic acid
alpha -hydroxydocosanoic acid
alpha -hydroxyhexacosanoic acid, and
alpha -hydroxyoctacosanoic acid

When the conditioning agent is an emollient it may be selected from hydrocarbons, fatty acids, fatty alcohols and esters. Isononyl isononanoate is the most preferred hydrocarbon type of emollient conditioning agent. Other hydrocarbons that may be employed include mineral oil, polyolefins such as polydecene, and paraffins such as isohexadecane (e.g. Permethyl 99 Registered TM and Permethyl 101 Registered TM). Preferably, the compositions of the present invention are substantially free of semi-solid hydrocarbons such as petrolatum, lanolin and lanolin derivatives, sterols (e.g., ethoxylated soya sterols), high molecular weight polybutenes and cocoa butter. By "substantially free," as used herein, means that the concentration of the semi-solid hydrocarbons are preferably less than 10%, more preferably less than 5% most preferably less than 2% and even more preferably 0. Without being limited by theory, such semi-solid hydrocarbons tend to mask the sensory benefits of the siloxane polymer gel network compositions such as the non-greasy, light feel of the present invention.

Fatty acids and alcohols will have from 10 to 30 carbon atoms. Illustrative of this category are pelargonic, lauric, myristic, palmitic, stearic, isostearic, hydroxystearic, oleic, linoleic, ricinoleic, arachidic, behenic and erucic acids and alcohols.

Oily ester emollients may be those selected from one or more of the following classes:
1. Triglyceride esters such as vegetable and animal fats and oils. Examples include castor oil, safflower oil, cottonseed oil, corn oil, olive oil, cod liver oil, almond oil, avocado oil, palm oil, sesame oil, squalene, Kikui oil and soybean oil.
2. Acetoglyceride esters, such as acetylated monoglycerides.
3. Ethoxylated glycerides, such as ethoxylated glyceryl monostearate.
4. Alkyl esters of fatty acids having 10 to 20 carbon atoms. Methyl, isopropyl, and butyl esters of fatty acids are useful herein. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
5. Alkenyl esters of fatty acids having 10 to 20 carbon atoms. Examples thereof include oleyl myristate, oleyl stearate, and oleyl oleate.
6. Ether-esters such as fatty acid esters of ethoxylated fatty alcohols.
7. Polyhydric alcohol esters. Ethylene glycol mono and di-fatty acid esters, diethylene glycol mono-and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol polyfatty esters, ethoxylated glyceryl monostearate, 1,2-butylene glycol monostearate, 1,2-butylene glycol distearate, polyoxyethylene polyol fatty acid ester, sorbitan fatty acid esters, and polyoxyethylene sorbitan fatty acid esters are satisfactory polyhydric alcohol esters.
8. Wax esters such as beeswax, spermaceti, myristyl myristate, stearyl stearate.
9. C1-C30 mono- and poly- esters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 1:3 molar ratio; and the octaester of sucrose wherein the esterifying carboxylic acid moieties are laurate, linoleate and behenate in a 1:3:4 molar ratio. A preferred solid material is sucrose polyester in which the degree of esterification is 7-8, and in which the fatty acid moieties are C18 mono- and/or di-unsaturated and behenic, in a molar ratio of unsaturates:behenic of 1:7 to 3:5. A particularly preferred solid sugar polyester is the octaester of sucrose in which there are about 7 behenic fatty acid moieties and about 1 oleic acid moiety in the molecule. Other materials include cottonseed oil or soybean oil fatty acid esters of sucrose. The ester materials are further described in, U.S. Patents 2,831,854; 4,005,196; 4,005,195; 5,306,516; 5,306,515; 5,305,514; 4,797,300; 3,963,699; 4,518,772; and 4,517,360.

Amounts of the skin-conditioning agent may range from about 0% to 30%, preferably from about 1% to about 20%, optimally from about 1% to 10% by weight of the composition.

### Solidifying Agent

The cosmetic compositions of this invention can contain one or more materials, herein singly or collectively referred to as a "solidifying agent", that are effective to solidify the particular liquid base materials to be used in a cosmetic composition. (As used herein, the term "solidify" refers to the physical and/or chemical alteration of the liquid base material so as to form a solid or semi-solid at ambient conditions, i.e., to form a final composition that has a stable physical structure and is deposited on the skin during normal use conditions.) As is appreciated by those skilled in the art, the selection of the particular solidifying agent for use in the cosmetic compositions will depend upon the particular type of composition desired, i.e., gel or wax-based, the desired rheology, the liquid base material used and the other materials to be used in the composition. The solidifying agent is preferably present at a concentration of from about 0 to about 90%, more preferably from about 1 to about 50%, even more preferably from about 5% to about 40%, most preferably from about 1% to about 15%.

Suitable solidifying agents include waxy materials such as candelilla, carnauba waxes, beeswax, spermaceti, carnauba, baysberry, montan, ozokerite, ceresin, paraffin, synthetic waxes such as Fisher-Tropsch waxes, silicone waxes (e.g., DC 2503 from Dow Corning), microcrystalline waxes and the like; soaps, such as the sodium and potassium salts of higher fatty acids, i.e., acids having from 12 to 22 carbon atoms; amides of higher fatty acids; higher fatty acid amides of alkylolamines; dibenzaldehyde-monosorbitol acetals; alkali metal and alkaline earth metal salts of the acetates, propionates and lactates; and mixtures thereof. Also useful are polymeric materials such as, locust bean gum, sodium alginate, sodium caseinate, egg albumin, gelatin agar, carrageenin gum sodium alginate, xanthan gum, quince seed extract, tragacanth gum, starch, chemically modified starches and the like, semi-synthetic polymeric materials such as cellulose ethers (e.g., hydroxyethyl cellulose, methyl cellulose, hydroxypropyl cellulose, carboxymethyl cellulose, hydroxy propylmethyl cellulose), polyvinylpyrrolidone, polyvinylalcohol, guar gum, hydroxypropyl guar gum, soluble starch, cationic celluloses, cationic guars and the like and synthetic polymeric materials such as carboxyvinyl polymers, polyvinylpynolidone, polyvinyl alcohol polyacrylic acid polymers, polymethaerylie acid polymers, polyvinyl acetate polymers, polyvinyl chloride polymers, polyvinylidene chloride polymers and the like. Inorganic thickeners may also be used such as aluminum silicates, such as, for example, bentonites, or a mixture of polyethylene glycol and polyethylene glycol stearate or distearate. Naturally occurring polymers or biopolymers and their use are further described in European Application No. 522624 invented by Dunphy et al. Additional examples of naturally occurring polymers or biopolymers can be found in the Cosmetic Bench Reference, pp. 1.40-1.42,

Also useful herein are hydrophilic gelling agents such as the acrylic acid/ethyl acrylate copolymers and the carboxyvinyl polymers sold by the B.F. Goodrich Company under the trademark of Carbopol Registered TM resins. These resins consist essentially of a colloidally water-soluble polyalkenyl polyether crosslinked polymer of acrylic acid crosslinked with from 0.75% to 2.00% of a crosslinking agent such as polyallyl sucrose or polyallyl pentaerythritol. Examples include Carbopol 934, Carbopol 940, Carbopol 950, Carbopol 980, Carbopol 951 and Carbopol 981. Carbopol 934 is a water-soluble polymer of acrylic acid crosslinked with about 1% of a polyallyl ether of sucrose having an average of about 5.8 allyl groups for each sucrose molecule. Also suitable for use herein are carbomers sold under the Trade Name "Carbopol Ultrez 10, Carbopol ETD2020, Carbopol 1382, Carbopol 1342 and Pemulen TR-1 (CTFA Designation: Acrylates/10-30 Alkyl Acrylate Crosspolymer). Combinations of the above polymers are also useful herein. Other gelling agents suitable for use herein include oleogels such as trihydroxystoarin.

Hydrophobically modified celluloses are also suitable for use herein. These celluloses are described in detail in U.S. Patents 4,228,277 and 5,104,646.

Additional examples of suitable gelling agents or gellants can be found in the Cosmetic Bench Reference, p. 1.27.

Further examples of suitable solidifying agents disclosed in the following references: U.S. Patents 4,151,272; 4,229,432; 4,280,994; "The Chemistry and Technology of Waxes", A. H. Warth, 2nd Edition, reprinted in 1960, Reinhold Publishing Corporation, pp 391-393 and 421; "The Petroleum Chemicals Industry", R. F. Goldstein and A. L. Waddeam, 3rd Edition (1967), E & F. N. Span Ltd., pp 33-40; "The Chemistry and Manufacture of Cosmetics", M. G. DeNavarre, 2nd edition ( 1970), Van Nostrand & Company, pp 354-376; and in "Encyclopedia of Chemical Technology:, Vol. 24, Kirk-Othmer, 3rd Edition (1979) pp 466-481; U.S. Patent 4,126,679;; European Patent Specification No. 117,070; U.S. Patents 2,900,306; 3,255,082; 4,137,306; 4,154,816; 4,226,889; 4,346,079; 4,383,988; European Patent Specification Nos. 107,330 and 24,365; and U.S. patent application Ser. No. 630,790.

### Additional Colorants

Certain embodiments of the present invention may optionally contain from about 0.1% to about 40%, preferably from about 1% to about 20%, more preferably from about 2% to about 15% and most preferably from about 4% to about 10%, of one or more additional colorants. Suitable colorants include the colorants that are discussed in the solid particle section, lakes, and dyes.

Colorants useful herein are all those pigments discussed earlier in the "solid particle" description, lakes, dyes, toners, and combinations thereof. Lakes are either a pigment that is extended or reduced with a solid diluent or an organic pigment that is prepared by the precipitation of a water-soluble dye on an adsorptive surface, which usually is aluminum hydrate. There is uncertainty in some instances as to whether the soluble dye precipitates on the surface of the aluminum hydrate to yield a dyed inorganic pigment or whether it merely precipitates in the presence of the substrate. A lake also forms from precipitation of an insoluble salt from an acid or basic dye. Calcium and barium lakes are also used herein.

### Preservatives

Suitable traditional preservatives for compositions of this invention are alkyl esters of para-hydroxybenzoic acid. Other preservatives that have more recently come into use include hydantoin derivatives such as 1,3-bis(hydroxymethyl)-5,5-dimthylhydantoin, propionate salts, and a variety of quaternary ammonium compounds such as benzalkonium chloride, quaternium 15 (Dowicil 200), benzethonium Chloride, and methylbenzethonium chloride. Cosmetic chemists are familiar with appropriate preservatives and routinely choose them to satisfy the preservative challenge test and to provide product stability. Particularly preferred preservatives are disodium EDTA, phenoxyethanol, methyl paraben, propyl paraben, imidazolidinyl urea (commercially available as Germall 1157), sodium dehydroacetate and benzyl alcohol. The preservatives should be selected having regard for the use of the composition and possible incompatibilities between the preservatives and other ingredients in the emulsion. Preservatives preferably are employed in amounts ranging from about 0% to about 5%, more preferably from about 0.01% to about 2.5%, and most preferably from about 0.01% to about 1%, by weight of the composition.

### Emulsifiers

The compositions of the present invention may optionally comprise one or more emulsifiers. These emulsifiers may be nonionic, anionic or cationic. Suitable emulsifiers are disclosed in, for example, U.S. Patents 3,755,560 and 4,421,769; and McCutcheon's Detergents and Emulsifiers, North American Edition, pages 317-324 (1986). Illustrative nonionic surfactants are alkoxylated compounds based on C10-C22 fatty alcohols and acids, and sorbitan. These materials are available, for instance, from the Shell Chemical Company under the Neodol trademark, copolymers of polyoxypropylene-polyoxyethylene, sold by the BASF Corporation under the Pluronic trademark, are sometimes also useful. Alkyl polyglycosides available from the Henkel Corporation may also be utilized for purposes of this invention. Anionic type emulsifiers or surfactants include fatty acid soaps, sodium lauryl sulphate, sodium lauryl ether sulphate, alkyl benzene sulphonate, mono- and di-alkyl acid phosphates and sodium fatty acyl isethionate. Amphoteric emulsifiers or surfactants include such materials as dialkylamine oxide and various types ofbetaines (such as cocamidopiopyl betaine).

Preferred for use herein are polyoxyalkylene copolymers also known as silicone polyethers. Polymers are described in detail in U. S. Patent 4,268,499. A particularly preferred polyoxyalkylene copolymer is known by its CTFA designation as dimethicones copolyol. A particularly preferred form of dimethicone copolyol is that supplied by Dow Coming as DC5225C.

The overall concentration of the emulsifier can be from 0% to about 10% of the formulation, preferably from 0.1% to about 5% and most preferably from about 0.1% to about 2%, by weight of the composition. Examples of suitable emulsifiers can be found in U.S. Patent 5,085,856; Japanese Patent Publication Sho 61-83110; European Patent Application EP 522624; U.S. patent 5,688,831; and Examples of suitable moistures can be found in Cosmetic Bench Reference, pp. 1.22, 1.24-1.26 (1996).

### Organic Sunscreens

Compositions of the present invention preferably comprise an organic sunscreen. Suitable sunscreens can have UVA absorbing properties, UVB absorbing properties or a mixture thereof. The exact amount of the sunscreen active will vary depending upon the desired Sun Protection Factor, i. e., the "SPF" of the composition as well as the desired level of UVA protection. The compositions of the present invention preferably comprise an SPF of at least 10, preferably at least 15. (SPF is a commonly used measure of photoprotection of a sunscreen against erythema. The SPF is defined as a ratio of the ultraviolet energy required to produce minimal erythema on protected skin to that required to products the same minimal erythema on unprotected skin in the same individual. See, Federal Register, 43, No 166, pp. 38206-38269, August 25, 1978). Compositions of the present invention preferably comprise from about 2% to about 20%, more typically from about 4% to about 14%, by weight, of organic sunscreen. Suitable sunscreens include, but are not limited to, those found in the CTFA International Cosmetic Ingredient Dictionary and Handbook, 7th edition, volume 2, pp. 1672, edited by Wenninger and McEwen (The Cosmetic, Toiletry, and Fragrance Association, Inc., Washington, D. C., 1997*).*

The compositions of the present invention preferably comprise a UVA absorbing sunscreen actives that absorb UV radiation having a wavelength of from about 320nm to about 400nm. Suitable UVA absorbing sunscreen actives are selected from dibenzoylmethane derivatives, anthranilate derivatives such as methylanthranilate and homomethyl, 1-N-acetylanthranilate, and mixtures thereof. Examples of dibenzoylmethane sunscreen actives are described in US Patent No 4,387,089 issued to Depolo; and in Sunscreens: Development, Evaluation, and Regulatory Aspects edited by N. J. Lowe and N. A. Shaath, Marcel Dekker, Inc (1990). The UVA absorbing sunscreen active is preferably present in an amount to provide broad-spectrum UVA protection either independently, or in combination with, other UV protective actives that may be present in the composition.

Preferred UVA sunscreen actives are dibenzoylmethane sunscreen actives and their derivatives. They include, but are not limited to, those selected from 2-methyldibenzoylmethane, 4-methyldibenzoylmethane, 4-isopropyldibenzoylmethane, 4-tert-butyldibenzoylmethane, 2, 4-dimethyldibenzoylmethane, 2, 5-dimethyldibenzoylmethane, 4, 4'-diisopropylbenzoylmethane, 4-(1, 1-dimethylethyl)-4'-methoxydibenzoylmethane, 2-methyl-5-isopropyl-4'-methoxydibenzoylmethane, 2-methyl-5-tert-butyl-4'-methoxy-dibenzoylmethane, 2, 4-dimethyl-4'-methoxydibenzoylmethane, 2, 6-dimethyl-4'-tert-butyl-4'methoxydibenzoylmethane, and mixtures thereof. Preferred dibenzoyl sunscreen actives include those selected from 4-(1, 1-dimethylethyl)-4'-methoxydibenzoylmethane, 4-isopropyldibenzoylmethane, and mixtures thereof. A more preferred sunscreen active is 4-(1, 1-dimethylethyl)-4'-methoxydibenzoylmethane.

The sunscreen active 4-(1, 1-dimethylethyl)-4'-methoxydibenzoylmethane, which is also known as butyl methoxydibenzoylmethane or Avobenzone, is commercially available under the names of Parsol® 1789 from Givaudan Roure (International) S. A. (Basel, Switzerland) and Eusolex® 9020 from Merck & Co., Inc (Whitehouse Station, NJ). The sunscreen 4-isoproplydibenzoylmethane, which is also known as isopropyldibenzoylmethane, is commercially available from Merck under the name of Eusolex® 8020.

The compositions of the present invention preferably further comprise a UVB sunscreen active that absorbs UV radiation having a wavelength of from about 290nm to about 320nm. The compositions preferably comprise an amount of the UVB sunscreen active that is safe and effective to provide UVB protection either independently, or in combination with, other UV protective actives that may be present in the compositions. The compositions preferably comprise from about 0.1% to abut 16%, more preferably from about 0.1% to about 12%, and most preferably from about 0.5% to about 8% by weight, of UVB absorbing organic sunscreen.

A wide variety of UVB sunscreen actives are suitable for use herein. Nonlimiting examples of such organic sunscreen actives are described in US Patent Nos. 5,087,372; 5,073,371; and 5,073,372. Preferred UVB sunscreen actives are selected from 2-ethylhexyl-2-cyano-3, 3-diphenylacrylate (referred to as octocrylene), 2-phenyl-benzimidazole-5-sulphonic acid (PBSA), cinnamates and their derivatives such as 2-ethylhexyl-p-methoxycinnamate and octyl-p-methoxycinnamate, TEA salicylate, octyldimethyl PABA, camphor derivatives and their derivatives, and mixtures thereof. Preferred organic sunscreen actives are 2-ethylhexyl-2-cyano-3, 3-diphenylacrylate (referred to as octocrylene), 2-phenyl-benzimidazole-5-sulphonic acid (PBSA), octyl-p-methoxycinnamate, and mixtures thereof. Salt and acid neutralised forms of the acidic sunscreens are also useful herein. When organic sunscreen salts, such as PBSA, are used within compositions of the present invention they can disrupt the action of the thickener with the result that the final product may have sub optimal rheology. This can be countered by the addition of higher levels of thickener, fatty alcohols or nonionic surfactants such that the rheology of the final product returns to the desired level.

An agent may also be added to any of the compositions useful in the present invention to stabilise the UVA sunscreen to prevent it from photo-degrading on exposure to UV radiation and thereby maintaining its UVA protection efficacy. Wide ranges of compounds have been cited as providing these stabilising properties and should be chosen to compliment both the UVA sunscreen and the composition as a whole. Suitable stabilising agents include, but are not limited to, those described in US Patents Nos 5,972,316; 5,968,485; 5,935,556; 5,827,508 and PCT patent publication WO 00/06110. Preferred examples of stabilising agents for use in the present invention include 2-ethylhexyl-2-cyano-3, 3-diphenylacrylate (referred to as octocrylene), ethyl-2-cyano-3, 3-diphenylacrylate, 2-ethylhexyl-3, 3-diphenylacrylate, ethyl-3, 3-bis(4-methoxyphenyl)acrylate, and mixtures thereof. 2-ethylhexyl-2-cyano-3, 3-diphenylacrylate is most preferred.

An agent may also be added to any of the compositions useful in the present invention to improve the skin substantivity of those compositions, particularly to enhance their resistance to being washed off by water, or rubbed off. A preferred agent that will provide this benefit is a copolymer of ethylene and acrylic acid. Compositions comprising this copolymer are disclosed in U.S. Patent 4,663,157.

### Inorganic Sunscreens

In addition to the organic sunscreens compositions of the present invention can additionally comprise inorganic physical sunblocks. Nonlimiting examples of suitable physical sunblocks are described in CTFA International Cosmetic Ingredient Dictionary, 6th Edition, 1995, pp. 1026-28 and 1103, Sayre, R. M. et al., "Physical Sunscreens", J. Soc. Cosmet. Chem., Vol 41, no 2, pp. 103-109 (1990). Preferred inorganic physical sunblocks are zinc oxide and titanium dioxide, and mixtures thereof.

When used, the physical sunblocks are present in an amount such that the present compositions are transparent on the skin (i.e., non-whitening), preferably less than or equal to about 5%. When titanium dioxide is used, it can have an anatase, rutile, or amorphous structure. Physical sunblock particles, e.g. titanium dioxide and zinc oxide, can be uncoated or coated with a variety of materials including but not limited to amino acids, aluminum compounds such as alumina, aluminum stearate, aluminum laurate, and the like; carboxylic acids and their salts e.g. stearic acid and its salts; phospholipids such as lecithin; organic silicone compounds; inorganic silicone compounds such as silica and silicates; and mixtures thereof. A preferred titanium dioxide is commercially available from Tayca (Japan) and is distributed by Tri-K Industries (Emerson, NJ) under the MT micro-ionized series (e.g., MT 100SAS).

The compositions of the present invention preferably comprise from about 0.1% to about 10%, more preferably from about 0.1% to about 4%, and most preferably from about 0.5% to about 2.5%, by weight, of inorganic sunscreen.

### Other Optional Ingredients

A variety of additional ingredients can be incorporated into the compositions of the present invention. Nonlimiting examples of these additional ingredients include additional skin care actives such as peptides (e.g., Matrixyl® [a pentapetide derivative]), farnesol, bisabolol, phytantriol, glycerol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B₃ (e.g., niacinamide) and vitamin B₅ (e.g., panthenol) and the like and mixtures thereof; sunscreens; anti-acne medicaments (resorcinol, salicylic acid, and the like; antioxidants (e.g., phytosterols, lipoic acid); flavonoids (e.g., isoflavones and phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol). Nonlimiting examples of suitable carboxylic copolymers, emulsifiers, emollients, and other additional ingredients are disclosed in U.S. Patents 5,011,681; 5,939,082.

### ASSOCIATED METHODS

Applicants have found that the compositions of the present invention are useful in a variety of applications directed to enhancement of mammalian skin. The methods of use for the compositions disclosed and claimed herein include, but are not limited to: 1) methods of increasing the substantivity of a cosmetic to skin; 2) methods of moisturizing skin; 3) methods of improving the natural appearance of skin, 4) methods of applying a color cosmetic to skin; 5) methods of preventing, retarding, and/or treating wrinkles; 6) methods of providing UV protection to skin; 7) methods of preventing, retarding, and/or controlling the appearance of oil; 8) methods of modifying the feel and texture of skin; 9) methods of providing even skin tone; 10) methods of preventing, retarding, and/or treating the appear of spider vessels and varicose veins; 11) methods of masking the appearance of vellus hair on skin; and 12) methods of concealing blemishes and/or imperfections in human skin, including acne, age spots, freckles, moles, scars, under eye circles, birth marks, post-inflammatory hyperpigmentation; 13) methods of enhancing or modifying skin color such as lightening, darkening, making more pink, making more yellow, making less dull, making less ashy, making less orange, making more radiant; 14) methods of artificial tanning; 15) methods of concealing vitiligo; 16) methods of concealing damage incurred to the skin as a result of trauma, e.g., cosmetic surgery, bums, stretching of skin, etc.; and 17) methods of concealing wrinkles, fie lines, pores, uneven skin surfaces, etc. Each of the methods discussed herein involve topical application of the claimed compositions to skin.

The following examples will more fully illustrate the embodiments of this invention. All parts, percentages and proportions referred to herein and in the appended claims are by weight unless otherwise indicated.

### EXAMPLES

The cosmetic products in the following examples illustrate specific embodiments of the cosmetic compositions of the present invention, but are not intended to be limiting thereof. All exemplified compositions can be prepared by conventional formulation and mixing techniques. Component amounts are listed as weight percents and may exclude minor materials such as diluents, filler, and so forth. The listed formulations, therefore, comprise the listed components and any minor materials associated with such components.

### Example I

A tinted moisturiser of the present invention is prepared as follows:

| **Ingredient** | **Wt%** |
|---|---|
| Colored crosslinked gel network¹ | 45.00 |
| Dimethicone copolyol crosspolymer (KSG21) | 5.00 |
| Cyclomethicone (DC245) | 24.25 |
| Propylparabens | 0.10 |
| Ethylparabens | 0.20 |
| Water | 15.00 |
| Glycerin | 10.00 |
| Benzyl alcohol | 0.25 |
| Methylparabens | 0.10 |
| Disodium EDTA | 0.10 |

| | |
|---|---|
| 1 - Colored crosslinked gel network comprising 5% pigments (titanium dioxide and iron oxides) having a 30 micron average particle size, approximately 12% polymer, and 83% cyclomethicone fluid. | |

In a suitable vessel, the water, glycerine, disodium EDTA and benzyl alcohol are added and mixed using conventional technology until a clear water phase is achieved. When water phase is clear add methylparabens and mix again until clear.

In a separate vessel, add the KSG21, DC245 and the parabens. This mixture is milled using a Silverson on high speed until homogeneous.

Next, the silicone phase and the clear, water phase are combined and milled using a Silverson on high speed until the water is fully incorporated and an emulsion is formed. The colored, crosslinked gel network is then added and the product is mixed again using a Silverson on high speed. The resulting finished product is then incorporated into the appropriate package.

### Examples II

A liquid foundation of the present invention is prepared as follows:

| **Ingredient** | **Wt%** |
|---|---|
| Colored crosslinked gel network¹ | 30.00 |
| Dimethicone copolyol and cyclopentasiloxane (DC5225C) | 10.00 |
| Cyclomethicone (DC245) | 9.13 |
| Propylparabens | 0.10 |
| Ethylparabens | 0.20 |
| Water | 24.00 |
| Titanium dioxide | 13.50 |
| Iron oxides | 2.50 |
| Glycerin | 10.00 |
| Benzyl alcohol | 0.25 |
| Methylparabens | 0.10 |
| Ammonium polyacrylate (Darvan 821A²) | 0.12 |
| Disodium EDTA | 0.10 |

| | |
|---|---|
| 1 - Colored crosslinked gel network comprising 10% pigments (titanium dioxide and iron oxides) having a 30 micron average particle size, approximately 12% polymer, and 78% cyclomethicone fluid. 2 - supplied by Vanderbilt | |

In a suitable vessel, the water, glycerine, disodium EDTA and benzyl alcohol are added and mixed using conventional technology until a clear water phase is achieved. When water phase is clear add methylparabens and mix again until clear. Then add the ammonium polyacrylate, titanium dioxide and iron oxides and mix to disperse. Mix the resultant phase with a Silverson SL2T or similar equipment on high speed (8,000 rpm, standard head) to fully deagglomerate the pigments.

In a separate vessel, add the DC5225C, DC245 and the parabens. This mixture is milled using a Silverson on high speed until homogeneous.

Next, the colored water phase and the clear, silicone phase are combined and milled using a Silverson on high speed until the water is fully incorporated and an emulsion is formed. Finally, the colored gel is then added and the product is mixed again using a Silverson on high speed. The resulting finished product is then incorporated into the appropriate package.

### Example III

A liquid foundation of the present invention is prepared as follows:

| **Ingredient** | **Wt%** |
|---|---|
| Colored crosslinked gel network¹ | 30.00 |
| Dimethicone copolyol crosspolymer (KSG21) | 5.00 |
| Cyclomethicone (DC245) | 23.13 |
| Hydrophobic titanium dioxide | 1.50 |
| Hydrophobic iron oxides | 0.50 |
| Propylparabens | 0.10 |
| Ethylparabens | 0.20 |
| Water | 24.00 |
| Titanium dioxide | 4.30 |
| Iron oxides | 0.70 |
| Glycerin | 10.00 |
| Benzyl alcohol | 0.25 |
| Methylparabens | 0.10 |
| Ammonium polyacrylate (Darvan 821A²⁾ | 0.12 |
| Disodium EDTA | 0.10 |

| | |
|---|---|
| 1 - Colored gel comprising 10% pigments (titanium dioxide and iron oxides) having a 30 micron average particle size, approximately 12% polymer, and 78% cyclomethicone fluid. 2 - supplied by Vanderbilt | |

In a suitable vessel, the water, glycerine, disodium EDTA and benzyl alcohol are added and mixed using conventional technology until a clear water phase is achieved. When water phase is clear add methylparabens and mix again until clear. Then add the ammonium polyacrylate, titanium dioxide and iron oxides and mix to disperse. Mix the resultant phase with a Silverson SL2T or similar equipment on high speed (8,000 rpm, standard head) to fully deagglomerate the pigments.

In a separate vessel, add the KSG21, hydrophobic titanium dioxide and iron oxides, DC245 and the parabens. This mixture is milled using a Silverson on high speed until homogeneous.

Next, the colored water phase and the colored, silicone phase are combined and milled using a Silverson on high speed until the water is fully incorporated and an emulsion is formed. Finally, the colored gel is then added and the product is mixed again using a Silverson on high speed. The resulting finished product is then incorporated into the appropriate package.

### Example IV

A multichromatic, liquid foundation of the present invention is prepared as follows:

| **Ingredient** | **Wt%** |
|---|---|
| Colored crosslinked gel network¹ | 40.00 |
| Dimethicone copolyol crosspolymer (KSG21) | 5.00 |
| Cyclomethicone (DC245) | 19.35 |
| Propylparabens | 0.10 |
| Ethylparabens | 0.20 |
| Water | 15.00 |
| Titanium dioxide | 8.25 |
| Iron oxides | 1.75 |
| Glycerin | 10.00 |
| Benzyl alcohol | 0.25 |
| Methylparabens | 0.10 |
| Ammonium polyacrylate (Darvan 821A²⁾ | 0.12 |
| Disodium EDTA | 0.10 |

| | |
|---|---|
| 1 - Colored gel comprising 10% pigments (titanium dioxide and iron oxides) having a 60 micron average particle size, approximately 12% polymer, and 78% cyclomethicone fluid. 2 - supplied by Vanderbilt | |

In a suitable vessel, the water, glycerine, disodium EDTA and benzyl alcohol are added and mixed using conventional technology until a clear water phase is achieved. When water phase is clear add methylparabens and mix again until clear. Then add the ammonium polyacrylate, titanium dioxide and iron oxides and mix to disperse. Mix the resultant phase with a Silverson SL2T or similar equipment on high speed (8,000 rpm, standard head) to fully deagglomerate the pigments.

In a separate vessel, add the KSG21, hydrophobic titanium dioxide and iron oxides, DC245 and the parabens. This mixture is milled using a Silverson on high speed until homogeneous.

Next, the colored water phase and the colored, silicone phase are combined and milled using a Silverson on high speed until the water is fully incorporated and an emulsion is formed. Finally, a colored gel is then chosen so as to be significantly different in colour to the blend of titanium dioxide and iron oxides. This is then added and the product is mixed again using a Silverson on high speed. The resulting multichromatic, finished product is then incorporated into the appropriate package.

### Example V

A multichromatic, tinted foundation of the present invention is prepared as follows:

| **Ingredient** | **Wt%** |
|---|---|
| Colored crosslinked gel network¹ | 22.50 |
| Colored crosslinked gel network² | 22.50 |
| Dimethicone copolyol crosspolymer (KSG21) | 5.00 |
| Cyclomethicone (DC245) | 24.25 |
| Propylparabens | 0.10 |
| Ethylparabens | 0.20 |
| Water | 15.00 |
| Glycerin | 10.00 |
| Benzyl alcohol | 0.25 |
| Methylparabens | 0.10 |
| Disodium EDTA | 0.10 |

| | |
|---|---|
| 1 - Colored gel comprising 5% pigments (titanium dioxide and iron oxides) having a 30 micron average particle size, approximately 12% polymer, and 83% cyclomethicone fluid. 2 - Colored gel as above but significantly different in colour. | |

In a suitable vessel, the water, glycerine, disodium EDTA and benzyl alcohol are added and mixed using conventional technology until a clear water phase is achieved. When water phase is clear add methylparabens and mix again until clear.

In a separate vessel, add the KSG21, DC245 and the parabens. This mixture is milled using a Silverson on high speed until homogeneous.

Next, the silicone phase and the clear, water phase are combined and milled using a Silverson on high speed until the water is fully incorporated and an emulsion is formed. The colored, crosslinked gels are then added and the product is mixed again using a Silverson on high speed. The resulting finished product is then incorporated into the appropriate package.

### Example VI

A lip colouring product of the present invention is prepared as follows:

| **Ingredient** | **Wt%** |
|---|---|
| Colored crosslinked gel network¹ | 15.00 |
| Dimethicone copolyol crosspolymer (KSG21) | 1.00 |
| Dimethicone copolyol and cyclopentasiloxane (DC5225C) | 5.00 |
| Silicone acrylates polymer and cyclopentasiloxane (SA70) | 16.00 |
| Cyclomethicone (DC245) | 11.75 |
| Hydrophobic titanium dioxide | 2.00 |
| Hydrophobic iron oxides | 1.50 |
| Organic pigment | 2.00 |
| Hydrophobic mica | 5.00 |
| Propylparabens | 0.10 |
| Ethylparabens | 0.20 |
| Water | 30.00 |
| Glycerin | 10.00 |
| Benzyl alcohol | 0.25 |
| Methylparabens | 0.10 |
| Disodium EDTA | 0.10 |

| | |
|---|---|
| 1 - Colored crosslinked gel comprising 10% red iron oxide having a 30 micron average particle size, approximately 12% polymer, and 78% cyclomethicone fluid. | |

In a suitable vessel, the water, glycerine, disodium EDTA and benzyl alcohol are added and mixed using conventional technology until a clear water phase is achieved. When water phase is clear add methylparabens and mix again until clear.

In a separate vessel, add the DC5225C, KSG21, SA70, hydrophobic titanium dioxide, iron oxides and mica, organic pigment, DC245 and the parabens. This mixture is milled using a Silverson on high speed until homogeneous.

Next, the colored water phase and the colored, silicone phase are combined and milled using a Silverson on high speed until the water is fully incorporated and an emulsion is formed. Finally, the colored gel is then added and the product is mixed again using a Silverson on high speed. The resulting finished product is then incorporated into the appropriate package.

### Example VII

A mousse foundation product of the present invention is prepared as follows:

| **Ingredient** | **Wt%** |
|---|---|
| Colored crosslinked gel network¹ | 50.00 |
| Dimethicone / vinyl dimethicone crosspolymer with cyclopentasiloxane (DC9040) | 10.00 |
| Cyclomethicone (DC245) | 11.75 |
| Hydrophobic titanium dioxide | 8.00 |
| Hydrophobic iron oxides | 2.00 |

| | |
|---|---|
| 1 - Colored crosslinked gel comprising 10% pigments (titanium dioxide and iron oxides) having a 30 micron average particle size, approximately 12% polymer, and 78% cyclomethicone fluid. | |

In a vessel, add hydrophobic titanium dioxide, iron oxides and DC245. This mixture is milled using a Silverson on high speed until homogeneous. The, the colored polymer gel network gel and the DC9040 are added and the product is mixed again using a Silverson on high speed. The resulting finished product is then incorporated into the appropriate package.

### Example VIII

A line blurring concentrate of the present invention is prepared as follows:

| **Ingredient** | **Wt%** |
|---|---|
| Colored crosslinked gel network¹ | 75.00 |
| Isoeicosane | 5.00 |
| Cyclomethicone (DC245) | 18.50 |
| C30-45 alkyl methyl siloxane (AMS C30 Wax) | 1.50 |

| | |
|---|---|
| 1 - Colored crosslinked gel comprising 10% silica of 65 micron average particle size, approximately 12% polymer, and 78% cyclomethicone fluid. | |

In a vessel, add hydrophobic titanium dioxide, iron oxides and DC245. This mixture is milled using a Silverson on high speed until homogeneous. The, the colored gel and the DC9040 are added and the product is mixed again using a Silverson on high speed. The resulting finished product is then incorporated into the appropriate package.

### Example IX

A mascara of the present invention is prepared as follows:

| Ingredient | Wt.% |
|---|---|
| Carnauba Wax | 3.00 |
| Glyceryl Monostearate¹ | 7.50 |
| White Beeswax | 3.75 |
| C18-C36 Triglycerides² | 5.50 |
| Hydrogenated Glycerol Rosinate³ | 0.15 |
| Paraffin Wax 118/125 | 2.25 |
| Paraffin Wax | 2.25 |
| DC9040 gel network⁴ | 70.65 |
| Stearic Acid 3X | 4.00 |
| Oleic Acid | 0.75 |
| Si methicone | 0.20 |
| Total | 100.00 |

| | |
|---|---|
| ¹ Available as Emerest 2400 available form Henkel/Emery ² Available as Syncrowax HGL-C available from Croda, Inc. ³ Available as Foral 105 available from Hercules, Inc. ⁴ Crosslinked gel network comprising 10% black iron oxide and of 10 micron average particle size, approximately 12% polymer, and 78% cyclomethicone fluid. | |

The waxes and fats are mixed in a vessel equipped with a heating source. The waxes and fats are heated and mixed at low speed using a conventional blender to liquify the mixture. The mixing is continued until the mixture is homogeneous. To the homogenous mixture is added the crosslinked gel. The mixing rate is increased to high and the pigments are mixed into the mixture for about 30-35 minutes until uniformly dispersed. The combined mixture is cooled to a temperature above the solidification point and is then poured into suitable containers.

### Example X

Prepare a skin cleansing article that contains the compositions of the present invention as follows. First, prepare a representative cleansing component by mixing the following components.

| **Component** | Wt% |
|---|---|
| Decylpolyglucose | 14.8 |
| Cocamidopropyl betaine | 14.8 |
| Sodium lauroyl sarcosinate | 14.8 |
| Colored crosslinked gel network¹ | 3.6 |
| PEG 14M | 1.8 |
| Polyquaternium-10 | 0.9 |
| Dex panthenol | 0.7 |
| Phenoxyethanol | 0.5 |
| Benzyl alcohol | 0.5 |
| Methylparaben | 0.45 |
| Propylparaben | 0.25 |
| Disodium EDTA | 0.2 |
| Water | 46.7 |

| | |
|---|---|
| 1 - Colored crosslinked gel compromising 10% pigments (Titanium dioxide and iron oxides) and of 30 micron average particle size., approximately 12% polymer, and 72% cyclomethicone fluid. | |

Next, the cleansing component is applied to one side of a first substrate layer by extruding it through a coating head continuously in four lines separated by a distance of 20 mm, 40 mm, and 20 mm respectively, measuring widthwise across the web, making a pair of parallel lines on each side of the web. The cleansing component is extruded at a rate to yield 4.4 grams of cleansing component per finished article. The substrate is a spunlace blend of 70% rayon and 30% PET fibers, bonded with a styrene-butadiene adhesive, which is hydroapertured to form holes about 2 mm in diameter and having a basis weight of about 70 gsm. A second substrate web which is an airlaid, lofty, low density batting is continuously fed over the first substrate placing it in contact with the surfactant layer. The batting comprises a blend of 30% 15 denier PET fibers, 35% 3 denier bicomponent fibers with PET core and PE sheath, and 35% 10 denier bicomponent fibers of the same core-sheath composition, and has a basis weight of about 100 grams per square meter (gsm). The webs are continuously fed to an ultrasonic sealer which seals a dot pattern comprising a grid of 4 mm diameter sealing points spaced evenly across the web. The web is cut into individual articles measuring about 120 mm x 160 mm rectangles with rounded corners, which has a total of about 51 sealing points per article.

### Example XI-XV

Prepare skin cleansing and conditioning articles that include the compositions of the present invention as follows. First, prepare a cleansing component that includes the following components:

| **Component** | Wt% |
|---|---|
| C16-18, 150EO Alcohol Ethoxylate (Empilan KM50*) | 22.0 |
| Cocamidopropyl betaine (Empigen BS)* | 20.0 |
| MEA Laureth-3 ethoxylate sulphate (Marlinat MEA) | 20.0 |
| Citric Acid anhydrous | 0.15 |
| Sodium Lauroyl sarcosinate | 20.0 |
| Propylene glycol | 17.85 |

| | |
|---|---|
| *available from Albright & Wilson | |

Heat the mixture to 70°C, stirring continuously until it has a paste like consistency. Cool to solidify until ready to use.

Next, prepare a representative conditioning component for articles that include representative compositions.

| **Component** | **XI** | **XII** | **XIII** | **XIV** | **XV** |
|---|---|---|---|---|---|
| | **Wt%** | **Wt%** | **Wt%** | **Wt%** | **Wt%** |
| Hydrophobic Phase: | | | | | |
| SEFA* cottonate | 1.65 | 2.65 | 12.5 | 12.5 | |
| SEFA* behenate | 0.35 | 0.35 | 8.0 | 8.0 | |
| Tribehenin | | | 6.0 | 6.0 | |
| Colored crosslinked gel network¹ | 3 | 2 | 3 | 4 | 1 |
| Petrolatum | | | 4.0 | 4.0 | 3.4 |
| Cocoa butter | | | | | 15.5 |
| C10-C30 Cholesterol/Lanosterol esters | | | 13.0 | 13.0 | |
| C30-C45 alkylmethicone² | | | | | |
| Polyglyceryl-4 isostearate (and) Cetyl dimethicone (and) Hexyl laurate³ | 5.0 | 5.0 | | | |
| PEG 30 dipolyhydroxystearate⁴ | | | 3.0 | | |
| Tetraglyceryl monostearate | | | | 2.1 | |
| Decaglyceryl dipalmitate | | | | 0.90 | |
| Ceresin wax | | | | | 5.5 |
| Beeswax | | | | | 7.0 |
| Lecithin, purified | | | | | 10.0 |
| 1-Monostearin | | | | | 10.0 |
| | | | | | |
| Hydrophilic Phase: | | | | | |
| Glycerin | 70.0 | 66.5 | 42.30 | 42.30 | 40.0 |
| Water | | 3.5 | | | 5.0 |
| PVM/MA decadiene crosspolymer⁵ | | | 0.25 | | |
| Sodium hydroxide (10% solution) | | | 0.25 | | |
| Gelatin | | | | | 2.6 |
| | | | | | |
| Active skin care ingredients: | | | | | |
| Panthenol | 20.0 | 10.0 | 2.50 | | |
| Nicotinamide | | 5.0 | 2.50 | 3.0 | |
| Urea | | 5.0 | 2.50 | 2.50 | |
| Allantoin | | | 0.20 | 0.20 | |
| Acetamidopropyl trimonium chloride | | | | 2.0 | |

| | | | | | |
|---|---|---|---|---|---|
| * SEFA is an acronym for sucrose esters of fatty acids 1- Colored crosslinked gel comprising 10% pigments (titanium dioxide and iron oxides) having a 30 micron average particle size, approximately 12% polymer, and 78% cyclomethicone fluid 2- Available as AMS-C30 from Dow Coming 3- Available as Abil WE-09 from Goldschmidt 4- Available as Arlacel P135 from ICI 5- Available as Stabileze 06 from ISP | | | | | |

Heat the hydrophobic phase to 70C, add the hydrophobic active skin care ingredients, and stir until homogenous. Premix the hydrophilic phase ingredients with the hydrophilic active skin care ingredients, heating gently if necessary to dissolve or disperse them. Add these slowly to the hydrophobic phase, continuing to stir. Homogenize (high shear mixer; ultrasonic homogenizer; or high pressure homogenizer such as Microfluidizer from Microfluidics Corp.). Apply immediately to substrate surface or cool rapidly to below room temperature in ice or ice water. Store in controlled environment, under nitrogen if needed for chemical stability.

### Example XVI

Prepare a skin cleansing and conditioning article that contains compositions representative of the invention. First, apply the cleansing component of Examples XI-XV to one side of a first substrate layer as a hot liquid (70-80°C) using an extrusion jetting head such that the coating is in the form of four lines separated by a distance of 10 mm, 60 mm, and 10 mm respectively, measuring widthwise across the web, making a pair of parallel lines on each side of the web. The substrate is a spunlace blend of 70% rayon and 30% PET fibers, bonded with a styrene-butadiene adhesive, which is hydroapertured to form holes about 2 mm in diameter and having a basis weight of about 70 gsm. A second substrate web that is an airlaid, lofty, low density batting is continuously fed over the first substrate placing it in contact with the surfactant layer. The batting comprises a blend of 30% 15 denier PET fibers, 35% 3 denier bicomponent fibers with PET core and PE sheath, and 35% 10 denier bicomponent fibers of the same core-sheath composition, and has a basis weight of about 100 grams per square meter (gsm). The webs are continuously fed to an ultrasonic sealer that seals a dot pattern comprising a grid of 4 mm diameter sealing points spaced evenly across the web. Two grams of the skin conditioning component of any one of Examples XI-XV is applied half to each side of the sealed article. The composition is applied as a hot liquid (60-70°C) using an extrusion jetting head to create two stripes of coating 5mm wide and 100mm long, 2 cm apart on each side of the article. The web is cut into individual articles measuring about 120 mm x 160 mm rectangles with rounded corners, which has a total of about 51 sealing points per article.

### Example XVII

A liquid foundation of the present invention is prepared as follows:

| **Ingredient** | **Wt%** |
|---|---|
| Colored crosslinked gel network¹ | 40.00 |
| Dimethicone copolyol crosspolymer (KSG21) | 4.50 |
| Dimethicone Copolyol & Cyclomethicone | 0.50 |
| Cyclomethicone (DC245) | 23.37 |
| Hydrophobic titanium dioxide | 1.50 |
| Hydrophobic iron oxides | 0.50 |
| Propylparabens | 0.10 |
| Ethylparabens | 0.20 |
| Water | 14.00 |
| Titanium dioxide | 4.30 |
| Iron oxides | 0.70 |
| Glycerin | 10.00 |
| Benzyl alcohol | 0.25 |
| Methylparabens | 0.10 |
| Ammonium polyacrylate (Darvan 821A²⁾ | 0.12 |
| Disodium EDTA | 0.10 |

| | |
|---|---|
| 1 - Colored crosslinked gel comprising 10% red iron oxide having a 30 micron average particle size, approximately 12% polymer, and 78% cyclomethicone fluid. | |

In a suitable vessel, the water, glycerine, disodium EDTA and benzyl alcohol are added and mixed using conventional technology until a clear water phase is achieved. When water phase is clear add methylparabens and mix again until clear. Then add the ammonium polyacrylate, titanium dioxide and iron oxides and mix to disperse. Mix the resultant phase with a Silverson SL2T or similar equipment on high speed (8,000 rpm, standard head) to fully deagglomerate the pigments.

In a separate vessel, add the DC5225C, KSG21, hydrophobic titanium dioxide and iron oxides, DC245 and the parabens. This mixture is milled using a Silverson on high speed until homogeneous.

Next, the colored water phase and the colored, silicone phase are combined and milled using a Silverson on high speed until the water is fully incorporated and an emulsion is formed. Finally, a colored gel is then added and the product is mixed again using a Silverson on high speed. The resulting finished product is then incorporated into the appropriate package.

### Example XVIII

A liquid foundation of the present invention is prepared as follows:

| **Ingredient** | **Wt%** |
|---|---|
| Colored crosslinked gel network¹ | 25.00 |
| Dimethicone copolyol crosspolymer (KSG21) | 2.50 |
| Dimethicone Copolyol & Cyclomethicone | 2.50 |
| Cyclomethicone (DC245) | 23.37 |
| Hydrophobic titanium dioxide | 1.50 |
| Hydrophobic iron oxides | 0.50 |
| Propylparabens | 0.10 |
| Ethylparabens | 0.20 |
| Water | 24.00 |
| Titanium dioxide | 7.03 |
| Iron oxides | 0.87 |
| Glycerin | 10.00 |
| Benzyl alcohol | 0.25 |
| Methylparabens | 0.10 |
| Ammonium polyacrylate (Darvan 821A²⁾ | 0.12 |
| Disodium EDTA | 0.10 |

| | |
|---|---|
| 1 - Colored crosslinked gel comprising 10% red iron oxide having a 30 micron average particle size, approximately 12% polymer, and 78% cyclomethicone fluid. | |

In a suitable vessel, the water, glycerine, disodium EDTA and benzyl alcohol are added and mixed using conventional technology until a clear water phase is achieved. When water phase is clear add methylparabens and mix again until clear. Then add the ammonium polyacrylate, titanium dioxide and iron oxides and mix to disperse. Mix the resultant phase with a Silverson SL2T or similar equipment on high speed (8,000 rpm, standard head) to fully deagglomerate the pigments.

In a separate vessel, add the DC5225C, KSG21, hydrophobic titanium dioxide and iron oxides, DC245 and the parabens. This mixture is milled using a Silverson on high speed until homogeneous.

Next, the colored water phase and the colored, silicone phase are combined and milled using a Silverson on high speed until the water is fully incorporated and an emulsion is formed. Finally, a colored gel is then added and the product is mixed again using a Silverson on high speed. The resulting finished product is then incorporated into the appropriate package.

## Claims

1. A personal care composition **characterized in that** said composition comprises a three dimensional gel polymeric network comprising:
a. a non-linear polymer being a polysiloxane that is a crosslinked organopolysiloxane polymer gel network selected from the group consisting of non-emulsifying polymer gel networks, emulsifying polymer gel networks, and combinations thereof;
b. a plurality of solid particles that are entrapped within said polymer during polymerization of said polymer, and
c. a solvent for said polymer which serves to suspend and swell the polymer; wherein said solid particles are selected from the group consisting of:
shine control agents selected from the group consisting of silicas, and silicates or carbonates that are formed by reaction of a carbonate or a silicate with the alkali (IA) metals, alkaline earth (IIA) metals or transition metals;
soft focus powders;
sunscreen powders;
pigments selected from the group consisting of titanium dioxide, zinc oxide, red iron oxide, yellow iron oxide, black iron oxide, ultramarine, iron oxide titanated mica, bismuth oxychloride, and combinations thereof;
colorants;
filler powders; and,
combinations thereof.

2. The composition of Claim 1, wherein said composition is an emulsion selected from the group consisting of water-in-oil emulsions, oil-in-water emulsions, water-in-silicone emulsions, and combinations thereof.

3. The composition of Claim 1, wherein said polymer gel network is contained within the oily phase of the emulsion.

4. The composition of Claim 1, where said composition further comprises at least one additional three dimensional gel polymeric network that further comprises:
a. a second polymer;
b. at least one second colorant that is entrapped within said second polymer during polymerization; and
c. a second solvent in which said second polymer is dispersed.

5. A personal care composition as defined in claim 1, wherein said three dimensional gel polymeric network comprises at least one first entrapped colorant, and wherein said composition further comprises at least one second colorant that is substantially similar to said first colorant, wherein said second colorant is dispersed within said composition but is not entrapped in said polymer and is separate and distinct from said network.

6. The composition of Claim 5, where said composition further comprises at least one additional three dimensional gel polymeric network that further comprises:
a. a second polymer;
b. at least one third colorant that is entrapped within said second polymer during polymerization; and
c. a second solvent in which said second polymer is dispersed.

7. A personal care composition as defined in claim 1, wherein said three dimensional gel polymeric network comprises at least one first entrapped colorant and wherein said composition further comprises at least one second colorant that is substantially different in color from said first colorant, wherein said second colorant is dispersed within said composition but is not entrapped in said polymer and is separate and distinct from said network.

8. The composition of Claim 7, where said composition further comprises at least one additional three dimensional gel polymeric network that further comprises:
a. a second polymer;
b. at least one third colorant that is entrapped within said second polymer during polymerization; and
c. a second solvent in which said second polymer is dispersed.

## Patentansprüche

1. Körperpflegezusammensetzung, **dadurch gekennzeichnet, dass** die Zusammensetzung ein dreidimensionales Polymergelnetzwerk umfasst, das Folgendes umfasst:
a. ein nicht lineares Polymer, das ein Polysiloxan ist, das ein vernetztes Organopolysiloxanpolymer-Gelnetzwerk ist, das ausgewählt ist aus der Gruppe bestehend aus nicht emulgierenden Polymergelnetzwerken, emulgierenden Polymergelnetzwerken und Kombinationen davon,
b. mehrere Feststoffteilchen, die in dem Polymer während der Polymerisation des Polymers eingeschlossen werden, und
c. ein Lösungsmittel für das Polymer, das zum Suspendieren und Quellen des Polymers dient,
wobei die Feststoffteilchen ausgewählt sind aus der Gruppe bestehend aus:
Glanzreguliermitteln, die ausgewählt sind aus der Gruppe bestehend aus Siliciumdioxiden und Silikaten oder Carbonaten, die durch die Reaktion eines Carbonats oder eines Silikats mit Alkalimetallen (IA), Erdalkalimetallen (IIA) oder Übergangsmetallen gebildet werden, Weichzeichnungspulvern,
Sonnenschutzpulvern,
Pigmenten, die ausgewählt sind aus der Gruppe bestehend aus Titandioxid, Zinkoxid, rotem Eisenoxid, gelbem Eisenoxid, schwarzem Eisenoxid, Ultramarin, titaniertem Eisenoxid-Glimmer, Bismutoxychlorid und Kombinationen davon,
Farbstoffen,
Füllmittelpulvern und
Kombinationen davon.

2. Zusammensetzung nach Anspruch 1, wobei die Zusammensetzung eine Emulsion ist, die ausgewählt ist aus der Gruppe bestehend aus Wasser-in-Öl-Emulsionen, Öl-in-Wasser-Emulsionen, Wasser-in-Silikon-Emulsionen und Kombinationen davon.

3. Zusammensetzung nach Anspruch 1, wobei das Polymergelnetzwerk in der Ölphase der Emulsion enthalten ist.

4. Zusammensetzung nach Anspruch 1, wobei die Zusammensetzung ferner mindestens ein zusätzliches dreidimensionales Polymergelnetzwerk umfasst, das ferner Folgendes umfasst:
a. ein zweites Polymer,
b. mindestens einen zweiten Farbstoff, der in dem zweiten Polymer während der Polymerisation eingeschlossen wird, und
c. ein zweites Lösungsmittel, in dem das zweite Polymer dispergiert ist.

5. Körperpflegezusammensetzung nach Anspruch 1, wobei das dreidimensionale Polymergelnetzwerk mindestens einen ersten eingeschlossenen Farbstoff umfasst und wobei die Zusammensetzung ferner mindestens einen zweiten Farbstoff umfasst, der dem ersten Farbstoff im Wesentlichen ähnlich ist, wobei der zweite Farbstoff in der Zusammensetzung dispergiert, jedoch nicht in dem Polymer eingeschlossen ist und von dem Netzwerk getrennt und verschieden ist.

6. Zusammensetzung nach Anspruch 5, wobei die Zusammensetzung ferner mindestens ein zusätzliches dreidimensionales Polymergelnetzwerk umfasst, das ferner Folgendes umfasst:
a. ein zweites Polymer,
b. mindestens einen dritten Farbstoff, der in dem zweiten Polymer während der Polymerisation eingeschlossen wird, und
c. ein zweites Lösungsmittel, in dem das zweite Polymer dispergiert ist.

7. Körperpflegezusammensetzung nach Anspruch 1, wobei das dreidimensionale Polymergelnetzwerk mindestens einen ersten eingeschlossenen Farbstoff umfasst und wobei die Zusammensetzung ferner mindestens einen zweiten Farbstoff umfasst, dessen Farbe sich von dem ersten Farbstoff im Wesentlichen unterscheidet, wobei der zweite Farbstoff in der Zusammensetzung dispergiert, jedoch nicht in dem Polymer eingeschlossen ist und von dem Netzwerk getrennt und verschieden ist.

8. Zusammensetzung nach Anspruch 7, wobei die Zusammensetzung ferner mindestens ein zusätzliches dreidimensionales Polymergelnetzwerk umfasst, das ferner Folgendes umfasst:
a. ein zweites Polymer,
b. mindestens einen dritten Farbstoff, der in dem zweiten Polymer während der Polymerisation eingeschlossen wird, und
c. ein zweites Lösungsmittel, in dem das zweite Polymer dispergiert ist.

## Revendications

1. Composition de soin personnel, **caractérisée en ce que** ladite composition comprend un réseau polymère de gel tridimensionnel comprenant :
a. un polymère non linéaire qui est un polysiloxane qui est un réseau de gel polymère organopolysiloxane réticulé choisi dans le groupe constitué de réseaux de gel polymères non émulsifiants, réseaux de gel polymères émulsifiants, et leurs combinaisons ;
b. une pluralité de particules solides qui sont piégées au sein dudit polymère durant la polymérisation dudit polymère, et
c. un solvant pour ledit polymère qui sert à suspendre et gonfler le polymère ;
dans laquelle lesdites particules solides sont choisies dans le groupe constitué de :
agents de contrôle de brillance choisis dans le groupe constitué de silices, et silicates ou carbonates qui sont formés par réaction d'un carbonate ou d'un silicate avec les métaux alcalins (IA), les métaux alcalino-terreux (IIA) ou les métaux de transition ;
des poudres de floutage ;
des poudres d'écran solaire ;
des pigments choisis dans le groupe constitué du dioxyde de titane, l'oxyde de zinc, l'oxyde de fer rouge, l'oxyde de fer jaune, l'oxyde de fer noir, le bleu d'outre-mer, le mica titané à l'oxyde de fer, l'oxychlorure de bismuth, et leurs combinaisons ;
des colorants ;
des poudres de charge ; et,
leurs combinaisons.

2. Composition selon la revendication 1, où ladite composition est une émulsion choisie dans le groupe constitué d'émulsions eau-dans-huile, émulsions huile-dans-eau, émulsions eau-dans-silicone, et leurs combinaisons.

3. Composition selon la revendication 1, dans laquelle ledit réseau de gel polymère est contenu au sein de la phase huileuse de l'émulsion.

4. Composition selon la revendication 1, où ladite composition comprend en outre au moins un réseau polymère de gel tridimensionnel supplémentaire qui comprend en outre :
a. un deuxième polymère ;
b. au moins un deuxième colorant qui est piégé au sein dudit deuxième polymère durant la polymérisation ; et
c. un deuxième solvant dans lequel ledit deuxième polymère est dispersé.

5. Composition de soin personnel selon la revendication 1, dans laquelle ledit réseau polymère de gel tridimensionnel comprend au moins un premier colorant piégé, et où ladite composition comprend en outre au moins un deuxième colorant qui est essentiellement similaire audit premier colorant, dans laquelle ledit deuxième colorant est dispersé au sein de ladite composition, mais n'est pas piégé dans ledit polymère et est indépendant et distinct dudit réseau.

6. Composition selon la revendication 5, où ladite composition comprend en outre au moins un réseau polymère de gel tridimensionnel supplémentaire qui comprend en outre :
a. un deuxième polymère ;
b. au moins un troisième colorant qui est piégé au sein dudit deuxième polymère durant la polymérisation ; et
c. un deuxième solvant dans lequel ledit deuxième polymère est dispersé.

7. Composition de soin personnel selon la définition de la revendication 1, dans laquelle ledit réseau polymère de gel tridimensionnel comprend au moins un premier colorant piégé, et où ladite composition comprend en outre au moins un deuxième colorant qui est essentiellement de couleur différente par rapport audit premier colorant, dans laquelle ledit deuxième colorant est dispersé au sein de ladite composition, mais n'est pas piégé dans ledit polymère et est indépendant et distinct dudit réseau.

8. Composition selon la revendication 7, où ladite composition comprend en outre au moins un réseau polymère de gel tridimensionnel supplémentaire qui comprend en outre :
a. un deuxième polymère ;
b. au moins un troisième colorant qui est piégé au sein dudit deuxième polymère durant la polymérisation ; et
c. un deuxième solvant dans lequel ledit deuxième polymère est dispersé.
